# EUROPEAN PATENT APPLICATION

(11) **EP 4 596 684 A1**
(43) Date of publication of application: **06.08.2025**
(21) Application number: 23872262.3
(22) Date of filing: 25.09.2023
(51) Int. Cl.: C12N 5/10, A61K 35/17, A61P 1/04, C07K 14/495, C12N 5/0783

(54) **INDUCED REGULATORY T CELLS CONTAINING CHIMERIC ANTIGEN RECEPTOR (CAR)**

(30) Priority: 26.09.2022 JP 2022152882
(71) Applicant: Regcell Co., Ltd., Kyoto-shi, Kyoto 603-8436 (JP); OSAKA UNIVERSITY, Suita-shi Osaka 565-0871 (JP)
(72) Inventor: MIKAMI, Norihisa, Kyoto-shi, Kyoto 603-8436 (JP); SAKAGUCHI, Shimon, Suita-shi, Osaka 565-0871 (JP); NARUMIYA, Shuh, Kyoto-shi, Kyoto 603-8436 (JP); KAWAKAMI, Ryoji, Kyoto-shi, Kyoto 603-8436 (JP)
(74) Representative: Weickmann & Weickmann PartmbB
(86) International application number: PCT/JP2023/034731
(87) International publication number: WO 2024/071039

(57) **Abstract**

The present disclosure provides a pharmaceutical composition for treating or preventing a T cell-associated disease, including an inducible regulatory T cell having high functionality and a stable immunosuppressive function, and also provides inducible regulatory T cells containing a chimeric antigen receptor (CAR). In the present disclosure, there is provided a pharmaceutical composition for treating or preventing a T cell-associated disease, the pharmaceutical composition containing, as an active ingredient, inducible regulatory T cells having at least one characteristic selected from the group consisting of CTLA4 positive, NT5E positive, ITGAE (CD103) positive, and AREG positive. In the present disclosure, inducible regulatory T cells containing a chimeric antigen receptor (CAR) are provided.

## Description

### Technical Field

The present disclosure relates to inducible regulatory T cells containing a chimeric antigen receptor (CAR), and pharmaceutical compositions for treating or preventing a T cell-associated disease.

### Background Art

As an important characteristic of the CD25 positive CD4 positive regulatory T cells intrinsic to the immune system, the transcription factor FoxP3 is specifically expressed, and the occurrence and differentiation and the suppression function of the regulatory T cells may be impaired by the defect or mutation of FoxP3. The regulatory T cells suppress the immune system by expression of various genes such as CTLA4 and IL-10 in addition to FoxP3. It is considered that epigenetic states such as DNA demethylation contribute to comprehensive gene expression control of regulatory T cells including stable expression of FoxP3, and they correlate with the phenotype of functional regulatory T cells.

### Summary of Invention

### Solution to Problem

The present inventors have studied whether a chimeric antigen receptor (CAR) can be introduced into inducible regulatory T cells developed by themselves, have completed the present disclosure by containing the chimeric antigen receptor, and have found that inducible regulatory T cells containing a chimeric antigen receptor (CAR) can be provided as an innovative medicine. When inducing regulatory T cells from human peripheral T cells, inducible regulatory T cells used in the present disclosure are produced by inducing stable inducible T cells from human peripheral T cells by stimulation using an anti-CD3 antibody, then subjecting the cells to dormant culture, culturing the cells by performing anti-CD3 antibody stimulation again, and further subjecting the cells to dormant culture, and have high expression of suppression molecules and a high suppression function.

In addition, the present inventors have found that such inducible regulatory T cells can further express and/or retain a chimeric antigen receptor (CAR), and have obtained inducible regulatory T cells containing a chimeric antigen receptor (CAR) that can be used as a medicine.

Accordingly, the present disclosure provides the following:

### (Item 1)

Inducible regulatory T cells containing a chimeric antigen receptor (CAR).

### (Item 2)

The inducible regulatory T cells of the above item, wherein the CAR is expressed in the inducible regulatory T cells.

### (Item 3)

The inducible regulatory T cells of any one of the above items, having at least one characteristic selected from the group consisting of CTLA4 positive, NT5E positive, ITGAE (CD103) positive, and AREG positive.

### (Item 4)

The inducible regulatory T cells of any one of the above items, having at least two characteristics selected from the group consisting of CTLA4 positive, NT5E positive, ITGAE (CD103) positive, and AREG positive.

### (Item 5)

The inducible regulatory T cells of any one of the above items, which is at least CTLA4 positive.

### (Item 6)

The inducible regulatory T cells of any one of the above items, wherein the CNS2 site of the FOXP3 gene is demethylated.

### (Item 7)

The inducible regulatory T cells of any one of the above items, which are CD4 positive or CD8 positive.

### (Item 8)

The inducible regulatory T cells of any one of the above items, obtained from or induced from a human peripheral blood T cell, or a human tissue-derived T cell.

### (Item 8A)

The inducible regulatory T cells of any one of the above items, wherein the CAR is a second generation or third generation CAR.

### (Item 8B)

The inducible regulatory T cells of any one of the above items, wherein the CAR is a second generation CAR.

### (Item 8C)

The inducible regulatory T cells of any one of the above items, wherein the CAR is a third generation CAR.

### (Item 9)

The inducible regulatory T cells of any one of the above items, wherein the inducible regulatory T cells are obtained by a method including:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

### (Item 10)

A cell population including T cells, wherein about 50% or more of the T cells of the cell population are inducible regulatory T cells of any one of the above items.

### (Item 11)

The cell population according to item 10, wherein about 80% or more of the T cells in the cell population are inducible regulatory T cells of any one of the above items.

### (Item 12)

The cell population of any one of the above items, wherein the T cells in the cell population are regulatory T cells.

### (Item 13)

The cell population of any one of the above items, wherein about 90% or more of the cell population is T cells.

### (Item A1)

A pharmaceutical composition including inducible regulatory T cells containing a chimeric antigen receptor (CAR).

### (Item A2)

The pharmaceutical composition of any one of the above items, wherein the CAR is expressed on the inducible regulatory T cell.

### (Item A3)

The pharmaceutical composition of any one of the above items, wherein the inducible regulatory T cells have at least one characteristic selected from the group consisting of CTLA4 positive, NT5E positive, ITGAE (CD103) positive, and AREG positive.

### (Item A4)

The pharmaceutical composition of any one of the above items, wherein the inducible regulatory T cells have at least two characteristics selected from the group consisting of CTLA4 positive, NT5E positive, ITGAE (CD103) positive, and AREG positive.

### (Item A5)

The pharmaceutical composition of any one of the above items, wherein the inducible regulatory T cells are at least CTLA4 positive.

### (Item A6)

The pharmaceutical composition of any one of the above items, wherein the CNS2 site of the FOXP3 gene of the inducible regulatory T cells are demethylated.

### (Item A7)

The pharmaceutical composition of any one of the above items, wherein the inducible regulatory T cells are CD4 positive or CD8 positive.

### (Item A8)

The pharmaceutical composition of any one of the above items, wherein the inducible regulatory T cells are obtained from or induced from human peripheral blood T cells or human tissue-derived T cells.

### (Item A8A)

The pharmaceutical composition of any one of the above items, wherein the CAR is a second generation or third generation CAR.

### (Item A8B)

The pharmaceutical composition of any one of the above items, wherein the CAR is a second generation CAR.

### (Item A8C)

The pharmaceutical composition of any one of the above items, wherein the CAR is a third generation CAR.

### (Item A9)

A pharmaceutical composition of any one of the above items, wherein
the inducible regulatory T cells are obtained by a method including:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

### (Item A10)

The pharmaceutical composition of any one of the above items, including a T cell population wherein about 50% or more of the cells in the T cell population are the inducible regulatory T cells.

### (Item A11)

The pharmaceutical composition of any one of the above items, including a T cell population wherein about 80% or more of the cells in the T cell population are the inducible regulatory T cells.

### (Item A12)

The pharmaceutical composition of any one of the above items, wherein the T cell population is a regulatory T cell population.

### (Item A13)

The pharmaceutical composition of any one of the above items, wherein about 90% or more of the cell population is T cells.

### (Item A14)

The pharmaceutical composition of any one of the above items, including the inducible regulatory T cells such that about 10⁸ to about 10⁹, or about 10⁷ cells/kg are administered per dose.

### (Item A15)

The pharmaceutical composition of any one of the above items for treating or preventing a T cell-associated disease.

### (Item A16)

The pharmaceutical composition of any one of the above items, wherein the T cell-associated disease includes an autoimmune disease, allergy, graft rejection, a graft versus host disease, an inflammatory disease, an infection, cancer, and ALS.

### (Item A17)

The pharmaceutical composition of any one of the above items, wherein the autoimmune disease includes systemic lupus erythematosus, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, ulcerative colitis, and cardiomyopathy.

### (Item A18)

The pharmaceutical composition of any one of the above items, wherein the pharmaceutical composition is administered by injection.

### (Item A19)

The pharmaceutical composition of any one of the above items, wherein when the pharmaceutical composition is administered to a patient, the pharmaceutical composition is additionally administered to a patient who exhibits no response or an insufficient response to the pharmaceutical composition.

### (Item A20)

The pharmaceutical composition of any one of the above items, wherein the pharmaceutical composition is additionally administered at least about 2 weeks after the first administration.

### (Item A21)

A therapeutic agent for a T-cell associated disease including inducible regulatory T cells of any one of the above items or a cell population of any one of the above items, wherein a diagnosis is made with respect to the T cell-associated disease in the diseased subject, and the appropriate CAR contained in the inducible regulatory T cells or the cell population is selected based on the diagnosis.

### (Item AA1)

A method of treating or preventing a disease, disorder or condition that can be treated with inducible regulatory T cells in a subject, including a step of administering to the subject an effective amount of the inducible regulatory T cells containing a chimeric antigen receptor (CAR).

### (Item AA2)

The method of any one of the above items, wherein the CAR is expressed on the inducible regulatory T cell.

### (Item AA3)

The method of any one of the above items, wherein the inducible regulatory T cells have at least one characteristic selected from the group consisting of CTLA4 positive, NT5E positive, ITGAE (CD103) positive, and AREG positive.

### (Item AA4)

The method of any one of the above items, wherein the inducible regulatory T cells have at least two characteristics selected from the group consisting of CTLA4 positive, NT5E positive, ITGAE (CD103) positive, and AREG positive.

### (Item AA5)

The method of any one of the above items, wherein the inducible regulatory T cells are at least CTLA4 positive.

### (Item AA6)

The method of any one of the above items, wherein the CNS2 site of the FOXP3 gene of the inducible regulatory T cells are demethylated.

### (Item AA7)

The method of any one of the above items, wherein the inducible regulatory T cells are CD4 positive or CD8 positive.

### (Item AA8)

The method of any one of the above items, wherein the inducible regulatory T cells are obtained from or induced from human peripheral blood T cells or human tissue-derived T cells.

### (Item AA8A)

The method of any one of the above items, wherein the CAR is a second generation or third generation CAR.

### (Item AA8B)

The method of any one of the above items, wherein the CAR is a second generation CAR.

### (Item AA8C)

The method of any one of the above items, wherein the CAR is a third generation CAR.

### (Item AA9)

The method of any one of the above items, further including a step of obtaining the inducible regulatory T cells are obtained by a method including:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

### (Item AA10)

The method of any one of the above items, including a T cell population wherein about 50% or more of the cells in the T cell population are the inducible regulatory T cells.

### (Item AA11)

The method of any one of the above items, including a T cell population wherein about 80% or more of the cells in the T cell population are the inducible regulatory T cells.

### (Item AA12)

The method of any one of the above items, wherein the T cell population is a regulatory T cell population.

### (Item AA13)

The method of any one of the above items, wherein about 90% or more of the cell population is T cells.

### (Item AA14)

The method of any one of the above items, including the inducible regulatory T cells such that about 10⁸ to about 10⁹, or about 10⁷ cells/kg are administered per dose.

### (Item AA15)

The method of any one of the above items, wherein the treatment or prevention of the disease, disorder or condition includes treatment or prevention of a T cell-associated disease.

### (Item AA16)

The method of any one of the above items, wherein the disease, disorder or condition includes an autoimmune disease, allergy, graft rejection, a graft versus host disease, an inflammatory disease, an infection, cancer, and ALS.

### (Item AA17)

The method of any one of the above items, wherein the autoimmune disease includes systemic lupus erythematosus, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, ulcerative colitis, and cardiomyopathy.

### (Item AA18)

The method of any one of the above items, wherein the administration includes administration by injection.

### (Item AA19)

The method of any one of the above items, including additionally administering the inducible regulatory T cells to a subject who exhibits no response or an insufficient response to the inducible regulatory T cells when administered to the subject.

### (Item AA20)

The method of any one of the above items, wherein the inducible regulatory T cells, if administered additionally, are administered additionally at least about 2 weeks after the first administration.

### (Item AA21)

The method further includes making a diagnosis of the disease, disorder or condition in the subject and selecting the appropriate CAR contained in the inducible regulatory T cells or the cell population based on the diagnosis.

### (Item AAA1)

Use of inducible regulatory T cells containing a chimeric antigen receptor (CAR) for the production of a medicine for the treatment or prevention of a disease, disorder or condition which may be treated by the inducible regulatory T cells.

### (Item AAA2)

The use of any one of the above items, wherein the CAR is expressed on the inducible regulatory T cell.

### (Item AAA3)

The use of any one of the above items, wherein the inducible regulatory T cells have at least one characteristic selected from the group consisting of CTLA4 positive, NT5E positive, ITGAE (CD103) positive, and AREG positive.

### (Item AAA4)

The use of any one of the above items, wherein the inducible regulatory T cells have at least two characteristics selected from the group consisting of CTLA4 positive, NT5E positive, ITGAE (CD103) positive, and AREG positive.

### (Item AAA5)

The use of any one of the above items, wherein the inducible regulatory T cells are at least CTLA4 positive.

### (Item AAA6)

The use of any one of the above items, wherein the CNS2 site of the FOXP3 gene of the inducible regulatory T cells are demethylated.

### (Item AAA7)

The use of any one of the above items, wherein the inducible regulatory T cells are CD4 positive or CD8 positive.

### (Item AAA8)

The use of any one of the above items, wherein the inducible regulatory T cells are obtained from or induced from human peripheral blood T cells or human tissue-derived T cells.

### (Item AAA8A)

The use of any one of the above items, wherein the CAR is a second generation or third generation CAR.

### (Item AAA8B)

The use of any one of the above items, wherein the CAR is a second generation CAR.

### (Item AAA8C)

The use of any one of the above items, wherein the CAR is a third generation CAR.

### (Item AAA9)

The use of any one of the above items, wherein
the inducible regulatory T cells are obtained by a method including:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

### (Item AAA10)

The use of any one of the above items, including a T cell population wherein about 50% or more of the cells in the T cell population are the inducible regulatory T cells.

### (Item AAA11)

The use of any one of the above items, including a T cell population wherein about 80% or more of the cells in the T cell population are the inducible regulatory T cells.

### (Item AAA12)

The use of any one of the above items, wherein the T cell population is a regulatory T cell population.

### (Item AAA13)

The use of any one of the above items, wherein about 90% or more of the cell population is T cells.

### (Item AAA14)

The use of any one of the above items, including the inducible regulatory T cells such that about 10⁸ to about 10⁹, or about 10⁷ cells/kg are administered per dose.

### (Item AAA15)

The use of any one of the above items, wherein the disease, disorder or condition includes a T cell-associated disease.

### (Item AAA16)

The use of any one of the above items, wherein the disease, disorder or condition includes an autoimmune disease, allergy, graft rejection, a graft versus host disease, an inflammatory disease, an infection, cancer, and ALS.

### (Item AAA17)

The use of any one of the above items, wherein the autoimmune disease includes systemic lupus erythematosus, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, ulcerative colitis, and cardiomyopathy.

### (Item AAA18)

The use of any one of the above items, wherein the use is administered by injection.

### (Item AAA19)

The use of any one of the above items, wherein when the use is administered to a patient, the use is additionally administered to a patient who exhibits no response or an insufficient response to the use.

### (Item AAA20)

The use of any one of the above items, wherein the pharmaceutical composition is additionally administered at least about 2 weeks after the first administration.

### (Item AAA21)

The use wherein the medicine is characterized by making a diagnosis with respect to the disease, disorder or condition and selecting an appropriate CAR contained in the inducible regulatory T cells or the cell population based on the diagnosis.

### (Item B1)

A method of producing inducible regulatory T cells containing a chimeric antigen receptor (CAR), the method including:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

### (Item B2)

The method of any one of the above items, wherein the introduction of the CAR gene is performed at least about twice in total in any of steps (a) to (d).

### (Item B3)

The method of any one of the above items, wherein the introduction of the CAR gene is performed at least once in step (a).

### (Item B4)

The method of any one of the above items, wherein the first basal medium includes at least one factor selected from the group consisting of an anti-CD3 antibody, TGF-β1, IL-2, retinoic acid, a CDK8 inhibitor, a CDK19 inhibitor, a CDK8/19 inhibitor, and ascorbic acid.

### (Item B5)

The method of any one of the above items, wherein the first basal medium includes an anti-CD3 antibody, TGF-β1, IL-2, retinoic acid, a CDK8 inhibitor, a CDK19 inhibitor, a CDK8/19 inhibitor, and ascorbic acid.

### (Item B6)

The method of any one of the above items, wherein the second basal medium includes at least one factor selected from the group consisting of an anti-CD3 antibody, TGF-β1, IL-2, retinoic acid, a CDK8 inhibitor, a CDK19 inhibitor, and a CDK8/19 inhibitor.

### (Item B7)

The method of any one of the above items, wherein the second basal medium includes an anti-CD3 antibody, TGF-β1, IL-2, retinoic acid, a CDK8 inhibitor, a CDK19 inhibitor, and a CDK8/19 inhibitor.

### (Item B8)

The method of any one of the above items, wherein step (a) stimulates the CD4-positive T cells or CD8-positive T cells with the first basal medium for about 3 days.

### (Item B9)

The method of any one of the above items, wherein step (b) includes dormant culturing the cells obtained in step (a) in the medium including IL-2 for at least about 2 days.

### (Item B10)

The method of any one of the above items, wherein step (c) stimulates the cells obtained in step (b) with the second basal medium for about 3 days.

### (Item B11)

The method of any one of the above items, wherein step (d) includes dormant culturing the cells obtained in step (c) in the medium including IL-2 for at least about 2 days.

### (Item B12)

Inducible regulatory T cells or a cell population including the inducible regulatory T cells generated by the method of any one of the above items.

In the present disclosure, it is intended that the one or more characteristics described above may be provided in further combination in addition to the specified combination. It should be noted that further embodiments and advantages of the present disclosure are appreciated by those skilled in the art upon reading and understanding the following detailed description as appropriate.

Note that characteristics and remarkable function effects of the present disclosure other than those described above are apparent to those skilled in the art with reference to the following embodiments and drawings of the invention.

### Advantageous Effects of Invention

The regulatory T cells of the present disclosure can cause a chimeric antigen receptor (CAR) to function efficiently, and can be used as highly functional regulatory T cells for the treatment and prevention of various immune diseases and inflammatory diseases such as autoimmune diseases.

In addition, according to the present disclosure, it is possible to perform gene introduction into the inducible regulatory T cells of the present disclosure in such a manner that the inducible regulatory T cells can function efficiently using a lentivirus. Therefore, not only a chimeric antigen receptor (CAR) gene but also any gene can be introduced into the inducible regulatory T cells by the same method.

### Brief Description of Drawings

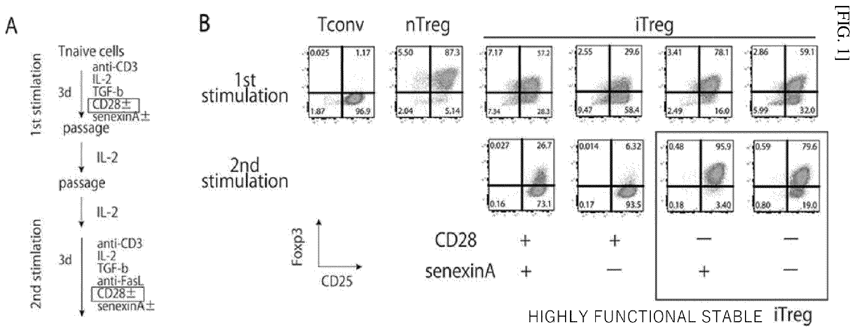

FIG. 1 shows an outline of a method for producing one embodiment of mouse inducible regulatory T cells (also referred to as mouse highly functional stable iTreg (HSF iTreg) cells) and results of flow cytometry on the inducible regulatory T cells. FIG. 1A shows a protocol outline when iTreg cells are induced from mouse CD4-positive naive T cells by various stimulation methods. FIG. 1B shows the results of analyzing the expression of FoxP3 and CD25 in iTreg cells, active T cells, and activated nTreg cells produced in FIG. 1A by a flow cytometry method.

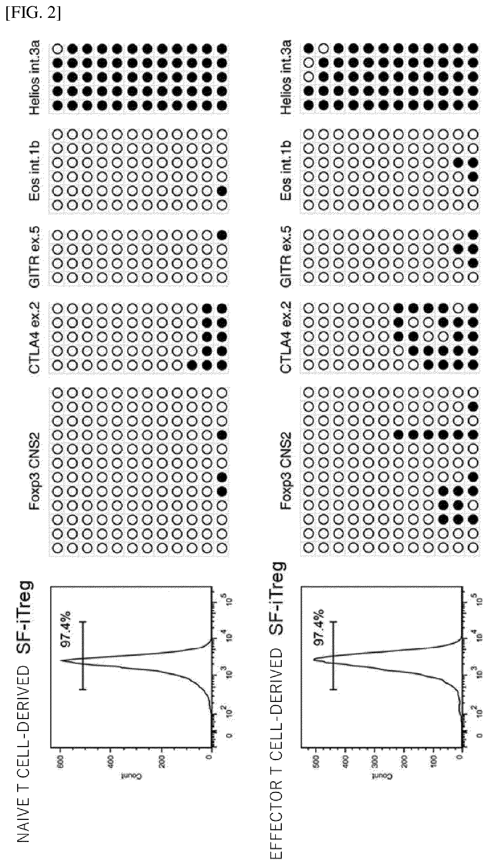

FIG. 2 is a diagram showing the results of analyzing FoxP3 expression and the demethylation state of a Treg-specific demethylated region by a flow cytometry method and a bisulfite method, respectively, when SF-iTreg is produced from mouse CD4 positive naive T cells or effector T cells on the basis of the production method according to one embodiment of iTreg(In the figure, SF-iTreg) of the present disclosure in FIG. 1.

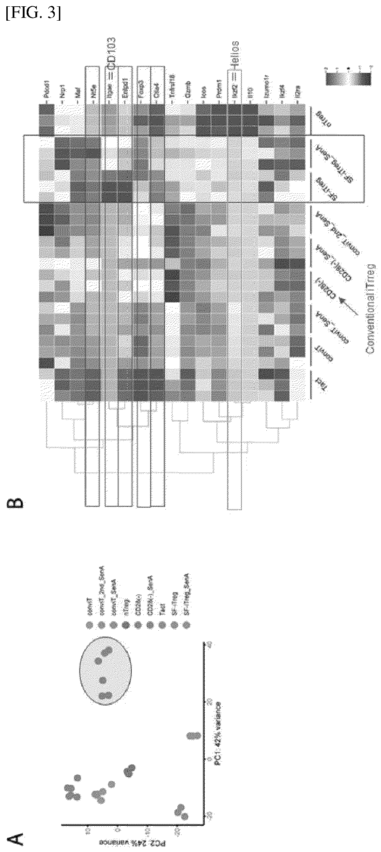

FIG. 3 shows a result of one embodiment in which a comprehensive gene expression pattern was analyzed for each cell in FIG. 1 by an RNA sequencing method. FIG. 3A shows a PCA analysis plot, and FIG. 3B shows a heat map of Treg related genes.

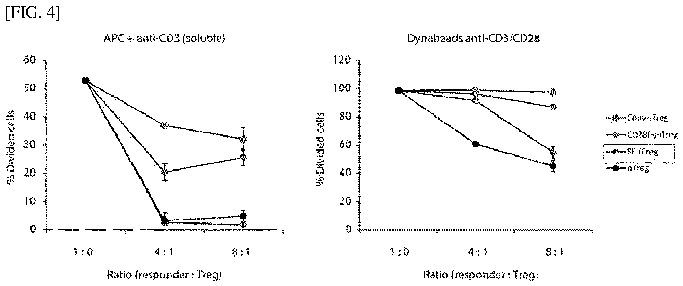

FIG. 4 is a result of one embodiment in which the in vitro suppression capacity of each cell in FIG. 1 was analyzed. Mouse CD4 positive naive T cells were stained with a Cell Trace Violet reagent, mixed cultured for 3 days in the presence of an anti-mouse CD3 antibody + MHC-II positive cells or Dynabeads T-activator (Veritas Corporation) (5 µL/well) at a constant ratio with each cell in FIG. 1, and Cell Trace Violet intensity was analyzed by a flow cytometry method.

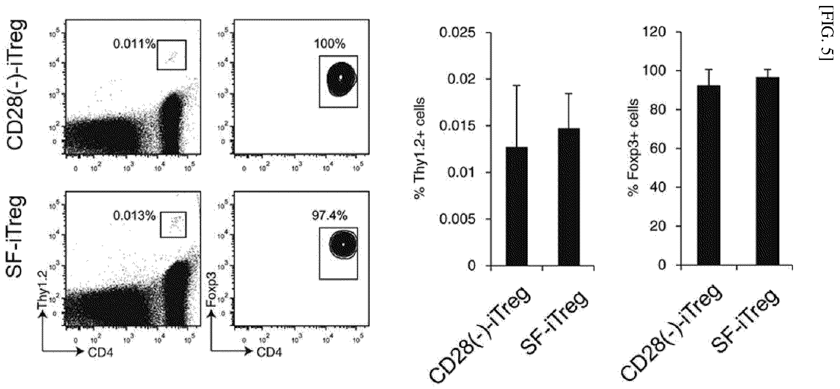

FIG. 5 is a result of one embodiment in which Foxp3 expression of regulatory T cells administered to a single stimulation CD28 antibody-unused group in FIG. 1 and iTreg of the present disclosure in a wild type mouse and transferred after 2 weeks was analyzed.

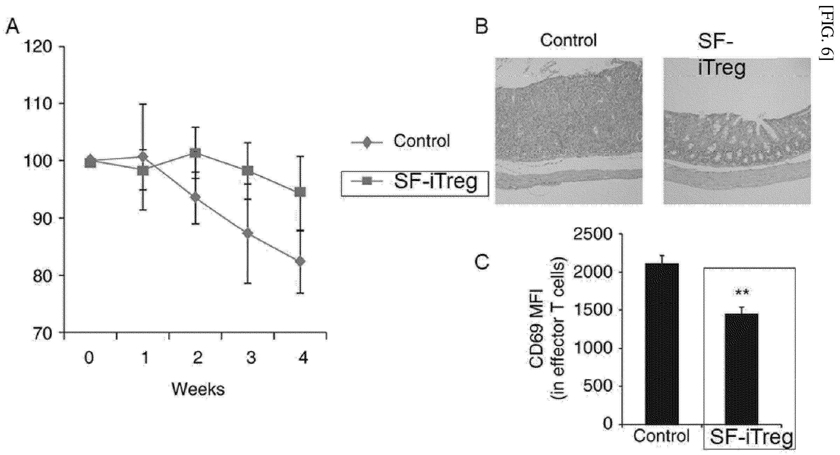

FIG. 6 is a result of one embodiment in which mouse CD4-positive naive T cells were transferred into a RAG2-deficient mouse to create a colitis model, and iTreg cells of the present disclosure were administered to analyze a treatment effect. FIG. 6A shows a change in body weight (g), FIG. 6B shows an HE staining image of a large intestine tissue, and FIG. 6C shows an analysis result of CD69 expression in lymph node T cells by a flow cytometry method.

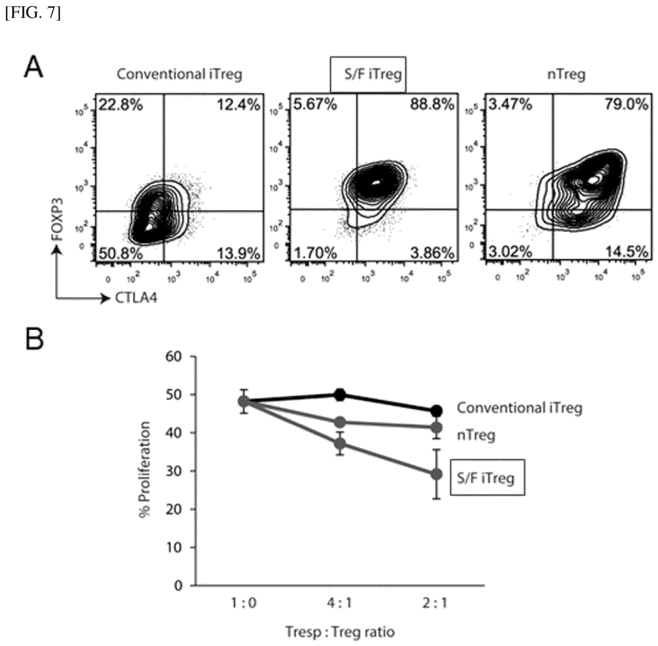

FIG. 7 is a diagram showing an analysis result of one embodiment of iTreg of the present disclosure derived from a human Crohn's disease patient. FIG. 7A shows a result of producing inducible regulatory T cells of the present disclosure (HSF-iTreg in the drawing) from CD4-positive T cells derived from a human Crohn's disease patient, and analyzing expression of FOXP3, CTLA4, and Helios by a flow cytometry method. Cells stimulated with normal nTreg (CD4 positive CD25 positive T cells) and normal iTreg (Conventional iTreg: cells in which CD4-positive T cells were stimulated with CD3/CD28 in the presence of IL-2 and TGF-β1 for 3 days) were compared. FIG. 7B is an analysis result of the in vitro suppression capacity of each cell of FIG. 7A. Human CD4-positive T cells were stained with a Cell Trace Violet reagent, mixed cultured for 3 days in the presence of a Treg Suppression Inspector (Miltenyi Biotec) (5 µL/well) at a constant ratio with each cell in FIG. 1, and the Cell Trace Violet intensity was analyzed by a flow cytometry method.

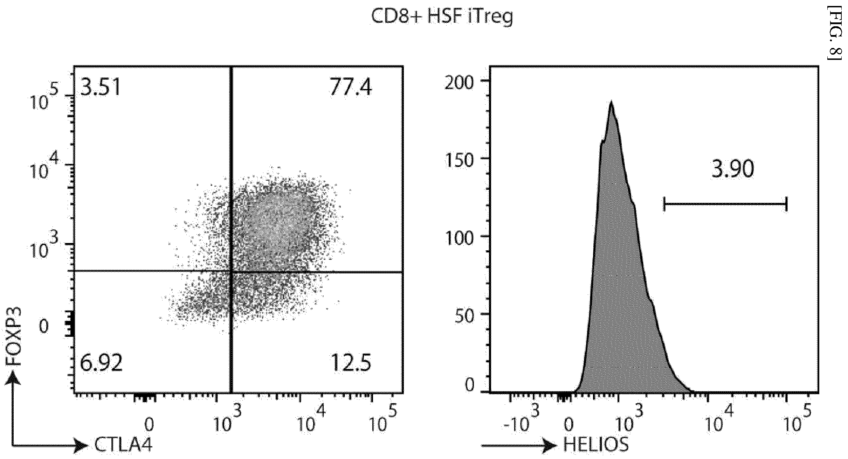

FIG. 8 shows phenotypic analysis (flow cytometry) of one embodiment of iTreg (HSF iTreg) cells of the present disclosure produced from human CD8-positive T cells. Highly functional inducible regulatory T cells were produced from human CD8-positive T cells, and the expression of FOXP3, CTLA4, and Helios was analyzed by flow cytometry.

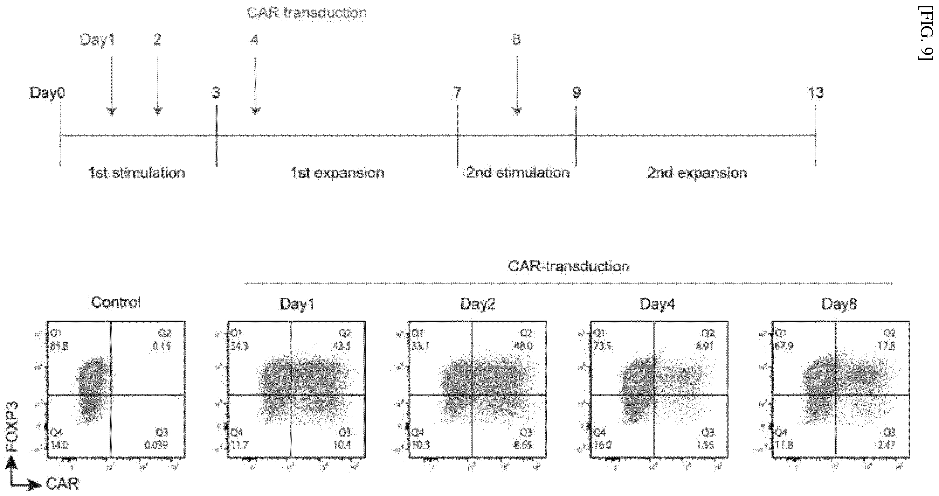

The schematic diagram shown in the upper part of FIG. 9 is a diagram showing the process of producing highly functional inducible regulatory T cells (HSF-iTreg) from human CD4-positive T cells and the timing of infecting a CAR-expressing viral vector-carrying lentivirus in the process. Lentivirus (Lenti-CD19 CAR (scFv-CD28, FMC63) Viral Particle, Cat. No.: VP-CAR-LC61) was added in an amount of 1/20 of the total amount of culture liquid at the timings of Day 1, Day 2, Day 4, and Day 8 as indicated by arrows, and FOXP3 and CAR expression were analyzed by FCM on the final day. The lower part of FIG. 9 shows FOXP3 and CAR gene expression on Day 13. It was shown that the CAR can be highly efficiently expressed by introducing the gene during the primary stimulation period.

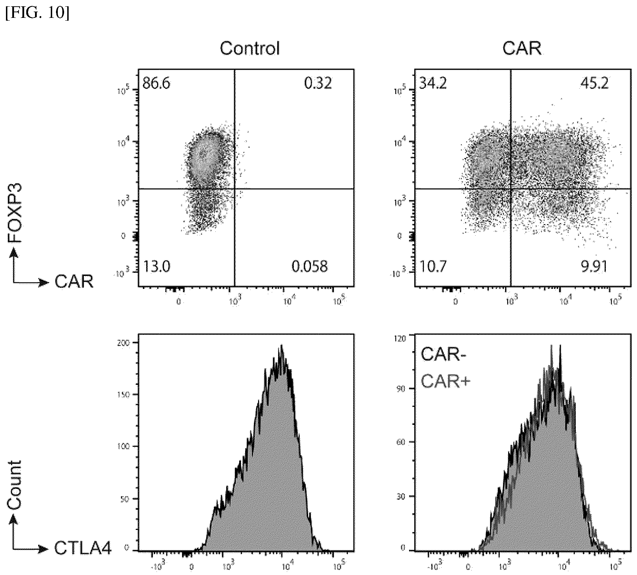

FIG. 10 shows a result of FCM analysis for expression of CTLA4 in a sample introduced on Day 1 of Example 7 in one embodiment of the present disclosure. High CTLA4 expression was also confirmed in the HSF-iTreg cell population expressing the CAR.

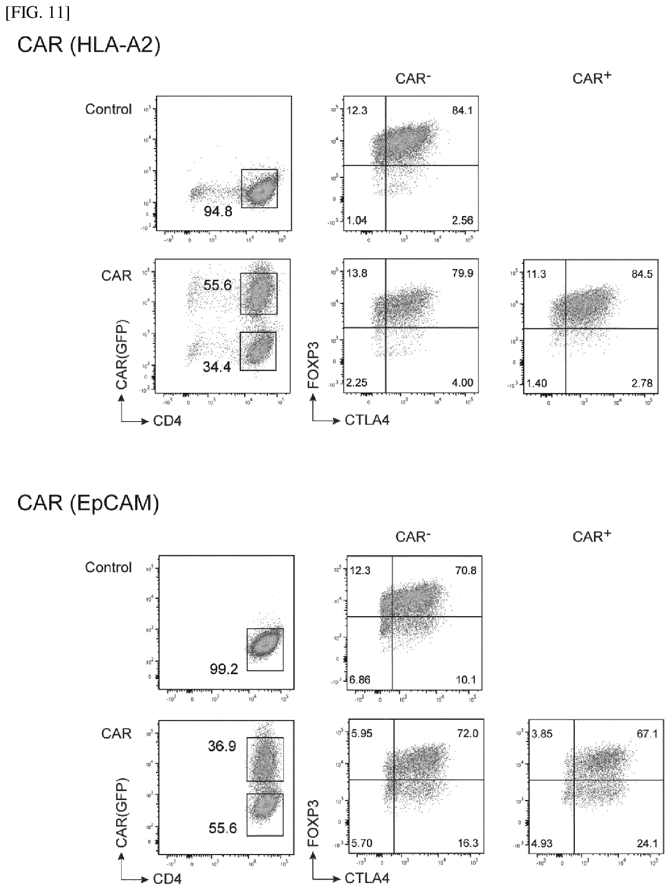

FIG. 11 shows the results of FCM analysis of the expression of CAR (GFP), FOXP3, and CTLA4 on the final day, with lentivirus (Lenti-HLA-A2 CAR (scFv-28ζ, BB7.2)-VP (VP-CAR-LC809) or Lenti-EpCAM CAR (scFv-28ζ, M13-57)-VP (VP-CAR-LC847)) added at the above timing in an amount of 1/50 or 1/25 of the total amount of culture liquid, respectively, in the process of producing highly functional inducible regulatory T cells (HSF-iTreg) from human CD4-positive T cells. It was shown that by introducing a gene during the primary stimulation period, any type of CAR can be highly efficiently expressed without impairing the properties of Treg and the induction efficiency. Description of Embodiments

Hereinafter, the present disclosure is described while showing the best mode. It is to be understood that throughout the present description, the singular forms of expression also include the concept of the plural forms thereof, unless otherwise stated. Thus, it is to be understood that the singular article (for example, in English, "a", "an", "the", and the like are used) also includes the plural concept thereof, unless otherwise stated. It is also to be understood that the terms used herein are used in the sense commonly used in the art, unless otherwise noted. Thus, unless defined otherwise, all technical and scientific terms used herein have the same meaning as commonly understood by one of ordinary skill in the art to which this disclosure belongs. In case of conflict, the present description (including definitions) will control.

Hereinafter, definitions and/or basic technical contents of terms particularly used in the present description are described as appropriate.

As used herein, "about" means ± 10% of the subsequent numerical value. For example, "about 20" includes "18 to 22". The range of numerical values includes all numerical values between the two end points and numerical values of the two end points. "About" with respect to a range applies to both endpoints of the range. Therefore, for example, "about 20 to 30" includes "18 to 33".

As used herein, when a gene name and a product thereof are expressed, they may refer to both a gene and a protein when expressed in all capital letters, unlike normal usage. For example, FOXP3 gene and FOXP3 protein may be used separately, and when expressed as FoxP3, both the concept and entity (whole) of the gene or protein are indicated.

As used herein, "regulatory T cells" are T cells positive for FoxP3 expression. As used herein, it may also be referred to as "Treg". Treg includes endogenous regulatory T cells (naturally occurring regulatory T cells: nTreg), inducible regulatory T cells (iTreg), and the like. Regulatory T cells usually can have a variety of functions (For example, immunosuppressive function).

As used herein, the term "inducible regulatory T cells (inducible regulatory T cells, iTreg)" refers to regulatory T cells whose expression of IKZF2 (Helios) is negative. iTreg is usually obtained by inducing differentiation from naive CD4-positive T cells or the like.

As used herein, the term "Endogenous regulatory T cells (naturally occurring regulatory T cells, nTreg)" refers to regulatory T cells that are positive for expression of IKZF2 (Helios) and CTLA4. Typically, these are cells exist in a living body.

As used herein, the term "peripheral T cells" refers to T cells existing outside the thymus, and can be obtained from peripheral blood, lymph nodes, or other tissues. As used herein, the term "peripheral T cells" only needs to include peripheral T cells in the cell group, and the T cells do not need to be isolated. In addition to T cells such as peripheral blood mononuclear cells (PBMC), cell fractions including various lymphocytes may be used.

As used herein, the term "flow cytometry" refers to a technique for measuring the number of particles of cells, individuals, and other biological particles suspended in a liquid, and the physical, chemical, and biological properties of each of the particles. A device using this technology is referred to as a "flow cytometer". In the present disclosure, the "positive" or "negative" of a cell marker (for example, FoxP3, CTLA4, Helios, CD103, etc.) is defined by flow cytometry, as commonly used in the art. More specifically, in flow cytometry, cells are flushed in a line and the number of cells is counted by spectroscopic techniques. For example, the number of target cells is counted by irradiating a cell labeled with fluorescence or a luminescent enzyme with laser light and detecting fluorescence or a luminescent signal emitted from the cell by a detector such as a photodiode. In addition, the detection result of the detector can be captured in a computer to generate and display a two-dimensional plot. Thereby, the existence of target cells, the number thereof, and the like can be easily grasped.

As used herein, "demethylation" refers to removal of methylation modification of adenine (For example, position 6; m6A, position 1; m1A) or cytosine (For example, position 5; m5C, position 3; m3C) which is typically methylated. Demethylation can be specified using a technique publicly known in the art, and can be measured using, for example, a bisulfite method.

As used herein, the "cell population" is a population including two or more cells, and for example, may be in a state where cells are gathered in a planar manner, or may be a cell mass configured by three-dimensionally adhering cells to each other. In addition, the "cell population" may be formed by a single type of cell or may include a plurality of types of cells.

As used herein, a "chimeric antigen receptor (CAR)" refers to a modified receptor capable of imparting antigen specificity to a cell (e.g., a Treg). CAR is also known as an artificial T cell receptor, a chimeric T cell receptor, or a chimeric immune receptor. Preferably, the CAR of the present disclosure includes at least one extracellular domain, at least one transmembrane domain, and at least one intracellular domain capable of binding an antigen. As used herein, when used in combination with T cells, may be referred to as CAR-T cells and the like. CAR that may be used include first, second and third generations, and the like. Here, the "first generation CAR" refers to a combination of a product (scFv) in which a VH chain and a VL chain of a monoclonal antibody variable region specific to a tumor-associated antigen are connected in series and a ζ chain of a T cell receptor. The "second generation CAR" refers to a first generation CAR that is incorporated with one co-stimulatory factor such as CD28 (related to PI3K) or 4-1BB (related to TRAFs), which is important for T-cell activation. The "third generation CAR" refers to a first generation CAR that is incorporated with a plurality of co-stimulatory factors such as CD28 (related to PI3K) or 4-1BB (related to TRAFs), which is important for T-cell activation.

As used herein, the term "T cell-associated disease" refers to any disease, disorder or condition in which the state of T cells is directly or indirectly associated. Therefore, T cell-associated diseases are expected to be treated or prevented by the inducible regulatory T cells of the present disclosure. Examples of the T cell-associated disease include an autoimmune disease, allergy, graft rejection, a graft versus host disease, an inflammatory disease, an infection, cancer, and ALS. In one embodiment, the autoimmune disease includes, but is not limited to, Addison's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, ulcerative colitis, cardiomyopathy, Dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, psoriasis, Raynaud's syndrome, preeclampsia, myocarditis, glaucoma, hypertension, pulmonary hypertension, malignant hypertension, Alzheimer's disease, systemic scleroderma, polymyositis, mixed connective tissue disease, antiphospholipid syndrome, microscopic polyangiitis, granulomatosis with polyangiitis, eosinophilic granulomatosis with polyangiitis, crescentic glomerulonephritis, organ-specific autoimmune disease, Graves' disease, autoimmune hepatitis, primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, sarcoidosis, and ulcerative colitis.

As used herein, "A disease, disorder or condition that can be treated with inducible regulatory T cells" includes, but is not limited to, any disease, disorder or condition for which regulatory T cells are generally effective even a little, including any disease, disorder or condition that may be treated or prevented, and it is understood that these are also encompassed because inducible regulatory T cells may be effective even when normal regulatory T cells are ineffective.

### (Preferred embodiment)

Preferred embodiments of the present disclosure are described below. The embodiments provided below are provided for a better understanding of the present disclosure, and the scope of the present disclosure should not be limited to the following description. Therefore, it is apparent that those skilled in the art can appropriately make modifications within the scope of the present disclosure in view of the description herein. In addition, the following embodiments of the present disclosure can be used alone or in combination thereof.

### <Inducible regulatory T cells containing chimeric antigen receptor (CAR) and use thereof>

In one aspect, the present disclosure provides inducible regulatory T cells containing a chimeric antigen receptor (CAR), and a method of producing, using, and other related techniques thereof.

The present disclosure utilizes highly functional stable inducible regulatory T cells (it is also referred to as highly functional stable iTreg or HSF iTreg).

In an aspect of the present disclosure, inducible regulatory T cells containing a chimeric antigen receptor (CAR) are provided as a pharmaceutical composition for treating or preventing. The inducible regulatory T cells used in the present disclosure have at least one characteristic selected from the group consisting of CTLA4 positive, NT5E positive, ITGAE (CD103) positive, and AREG positive. In one embodiment of the disclosure, inducible regulatory T cells of the disclosure can also have at least two characteristics selected from the group consisting of CTLA4 positive, NT5E positive, ITGAE (CD103) positive, and AREG positive. In one embodiment of the disclosure, the inducible regulatory T cells in the disclosure may also be at least CTLA4 positive. Without intending to be limiting, inducible regulatory T cells in the present disclosure may be CD4 positive or CD8 positive.

In one embodiment, the CAR is expressed in inducible regulatory T cells. In one embodiment, the CAR is one in which the gene is contained in inducible regulatory T cells. The CAR used may be in any form and any of the first, second and third generations may be suitably utilized. Without wishing to be bound by theory, as used in the present disclosure, it may be advantageous, but not limited to, having an appropriate stimulation intensity due to the relative relationship to the subject iTreg. From this point of view, second or third generation may be preferred, but not limited thereto. In one embodiment, the second generation may be advantageous, and in another embodiment, the third generation may be advantageous. The compatibility with the iTreg may be separately examined and selected according to an appropriate stimulation intensity (for example, the nutritional metabolic state, the intensity of phosphorylation, and the like may be measured in vitro as an index of stimulation intensity, and the resulting FOXP3 expression stability may be measured in vitro or in vivo), but the present disclosure is not limited thereto.

The expression of FoxP3 in the regulatory T cells can be induced in a test tube by culturing CD4-positive T cells in a medium including IL2 and TGFβ in the presence of an anti-CD3 antibody and an anti-CD28 antibody, and then culturing in a medium including IL2 and TGFβ. In addition, although some methods for inducing regulatory T cells from peripheral T cells are known, expression of FoxP3 in inducible regulatory T cells is unstable, and expression of many functional molecules other than FoxP3 has not been confirmed.

In recent years, a culture method for inducing DNA demethylation by a method in which ascorbic acid is added to a medium in inducible regulatory T cells (Kasahara et al. Int. Immunol. (2017) 29 (10): 457-469) and a method in which CD28 antibody stimulation is not used (Mikami et al. Proc Natl Acad Sci U S A. (2020) 117 (22): 12258-12268.) has been developed. However, expression of functional molecules has not been confirmed even in inducible regulatory T cells produced by this method, and sufficient immunosuppressive activity has not been obtained. Also in clinical trials, one clinical trial using inducible regulatory T cells has been conducted for GVHD, but the percentage of regulatory T cells used is low, and efficacy such as prevention of GVHD has not been recognized at present (MacMillan et al., Blood Adv. (2021) 5 (5): 1425-1436).

In the present disclosure, it is possible to provide a pharmaceutical composition containing inducible regulatory T cells as an active ingredient in which expression of FoxP3 is stable and advantageously stably retains an immunosuppressive effect, and such inducible regulatory T cells can be produced, for example, by a method for producing inducible regulatory T cells described elsewhere in the present description. For example, the present disclosure can provide a pharmaceutical composition wherein the inducible regulatory T cells are obtained by a method including: (a) a step of stimulating CD4-positive T cells or CD8-positive T cells in peripheral blood with a first basal medium for about 1 to about 5 days; (b) a step of dormant culturing cells obtained in step (a) in a medium including IL-2 for at least about 1 to about 3 days; (c) a step of stimulating cells obtained in step (b) with a second basal medium for about 1 to about 5 days; and (d) a step of dormant culturing cells obtained in step (c) in a medium including IL-2 for at least about 1 to about 3 days. The inducible regulatory T cells containing a chimeric antigen receptor (CAR) of the disclosure can be generated in such methods by including the step of introducing a CAR.

The inducible regulatory T cells in the present disclosure have an inducible regulatory T cell specific demethylation state. The fact that the obtained regulatory T cells are in a regulatory T cell-specific demethylated state can be confirmed, for example, by the fact that the CNS2 site of the gene of FoxP3 (FOXP3 (all in italics)) is demethylated. Since such a demethylated state can be an index of a stable type, the inducible regulatory T cells in the present disclosure can be shown to be stable inducible regulatory T cells by confirming the demethylated state.

As used herein, the immunosuppressive activity or immunosuppressive effect of inducible regulatory T cells in the present disclosure can be confirmed, for example, by measuring Cell Trace Violet intensity in responder T cells. The inducible regulatory T cells in the present disclosure can stably provide an immunosuppressive activity or an immunosuppressive effect, for example, the inducible regulatory T cells of the present disclosure can provide an immunosuppressive activity or an immunosuppressive action for at least about 2 weeks. As shown in the Examples below, inducible regulatory T cells in the present disclosure can have a higher immunosuppressive effect than conventional regulatory T cells, including inducible and endogenous T cells. Therefore, the inducible regulatory T cells in the present disclosure can also be referred to as functional or highly functional inducible regulatory T cells.

In one embodiment, inducible regulatory T cells in the present disclosure are capable of stably expressing FoxP3. Therefore, the inducible regulatory T cells in the present disclosure can also be referred to as stable inducible regulatory T cells. In one aspect, the inducible regulatory T cells in the present disclosure are highly functional and stable, and can be referred to as highly functional stable inducible regulatory T cells (HSF iTreg).

In one embodiment of the present disclosure, whether or not a marker in the inducible regulatory T cells of the present disclosure is positive can be determined by positive rate measurement by flow cytometry. For example, it is possible to determine whether or not the cells are positive from the proportion of cells showing an antigen expression level equal to or higher than a reference level by analyzing the cells with a flow cytometer. It can also be classified into negative, weakly positive, moderately positive, strongly positive (weakly positive, moderately positive, and strongly positive are combined to be "positive"), and the like according to the expression intensity of the cell surface marker. For example, depending on the setting of the instrument, when the value of the median fluorescence intensity of each marker/median fluorescence intensity of the negative control (staining with an isotype control antibody) is less than 5, 5 or more and less than 10, 10 or more and less than 30, or 30 or more, it can be determined as negative, weakly positive, moderately positive, or strongly positive, respectively. Such determinations can be made as exemplified in (positive rate measurement by flow cytometry), but the present disclosure is not limited thereto.

In one embodiment of the present disclosure, the inducible regulatory T cells in the present disclosure can be induced from any cell, and preferably can be induced from human peripheral blood T cells or human tissue-derived T cells.

In one embodiment of the disclosure, the disclosure can include a T cell population in which about 50% or more of the cells in the T cell population including a chimeric antigen receptor (CAR) are inducible regulatory T cells as described elsewhere herein. In one embodiment, the cell population of the disclosure can be about 60% or more, about 65% or more, about 70% or more, about 75% or more, about 80% or more, about 85% or more, about 90% or more, about 95% or more, about 97% or more, or about 99% or more of the T cells in the cell population are inducible regulatory T cells as described elsewhere in the present description.

In one embodiment of the disclosure, the T cells in the cell population of the disclosure can be regulatory T cells. In this case, about 60% or more, about 65% or more, about 70% or more, about 75% or more, about 80% or more, about 85% or more, about 90% or more, about 95% or more, about 97% or more, or about 99% or more of the regulatory T cells of the cell population of the present disclosure can be inducible regulatory T cells as described elsewhere in the present description.

In one embodiment of the disclosure, the cell population of the disclosure may include cells other than T cells, and preferably about 60% or more, about 65% or more, about 70% or more, about 75% or more, about 80% or more, about 85% or more, about 90% or more, about 95% or more, about 97% or more, or about 99% or more of the cell population of the disclosure may be T cells, and preferably about 90% or more may be T cells.

In an aspect of the disclosure, there is provided a pharmaceutical composition including inducible regulatory T cells or a cell population including a chimeric antigen receptor (CAR) of the disclosure. In yet another aspect, a regenerative medical material or product including inducible regulatory T cells or a cell population of the present disclosure is provided. This pharmaceutical composition or regenerative medical material or product can be used in an autoimmune disease, an inflammatory disease, and an allergy. These medicines, regenerative medical materials or products can be used with media or any other additive used in the art. As such a medium, a medium obtained by adding a necessary factor to a basal medium for culturing animal cells used for culturing cells can be used. Examples of such a medium are detailed elsewhere herein. Examples of components added to the medium are also detailed elsewhere herein. When provided as such a product, DMSO or the like may be included.

In one embodiment, the pharmaceutical compositions of the present disclosure can be used to treat or prevent T cell-associated diseases, which can include an autoimmune disease, allergy, graft rejection, a graft versus host disease, an inflammatory disease, an infection, cancer, ALS, and the like. In one embodiment, the autoimmune disease includes, but is not limited to, Addison's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, ulcerative colitis, cardiomyopathy, Dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, psoriasis, Raynaud's syndrome, preeclampsia, myocarditis, glaucoma, hypertension, pulmonary hypertension, malignant hypertension, Alzheimer's disease, systemic scleroderma, polymyositis, mixed connective tissue disease, antiphospholipid syndrome, microscopic polyangiitis, granulomatosis with polyangiitis, eosinophilic granulomatosis with polyangiitis, crescentic glomerulonephritis, organ-specific autoimmune disease, Graves' disease, autoimmune hepatitis, primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, sarcoidosis, and ulcerative colitis.

In one embodiment, the pharmaceutical compositions of the present disclosure can be administered by a variety of dosage and administration, but are preferably administered by injection. In one embodiment, a pharmaceutical composition of the present disclosure can include such that the inducible regulatory T cells described elsewhere herein are administered at about 10⁸ to about 10⁹, or about 10⁷/kg per dose.

In one embodiment, the pharmaceutical composition of the present disclosure, when administered to a patient, can also be additionally administered to a patient who exhibits no response or an insufficient response to the pharmaceutical composition. Whether the patient exhibits no response or an insufficient response can be determined by using, for example, the degree of suppression of inflammation in the target disease as an index. In one embodiment, if additional administration occurs, it can be administered at least about 1 week, about 2 weeks, about 3 weeks, or about 4 weeks after the first administration.

### <Method for producing inducible regulatory T cells>

The method for producing inducible regulatory T cells in the present disclosure is a novel method capable of producing highly functional and stable inducible regulatory T cells, and is described herein in detail below.

In an aspect of the present disclosure, there is provided a method of producing inducible regulatory T cells, the method including: (a) a step of stimulating CD4-positive T cells or CD8-positive T cells in peripheral blood with a first basal medium for about 1 to about 5 days; (b) a step of dormant culturing cells obtained in step (a) in a medium including IL-2 for at least about 1 to about 3 days; (c) a step of stimulating cells obtained in step (b) with a second basal medium for about 1 to about 5 days; and (d) a step of dormant culturing cells obtained in step (c) in a medium including IL-2 for at least about 1 to about 3 days. In the present disclosure, the inducible regulatory T cells in the present disclosure can be produced by using any of CD4-positive T cells and CD8-positive T cells as raw materials, and the CAR can be introduced by including the step of (e) introducing a chimeric antigen receptor (CAR) into the T cells. In one embodiment, the inducible regulatory T cells in the present disclosure can also be produced using mixed cells of CD4-positive T cells and CD8-positive T cells as raw materials.

In one embodiment, the methods of the present disclosure can also be methods of producing regulatory T cells from human peripheral T cells (including CD4-positive T cells or CD8-positive T cells) including a step of culturing human peripheral T cells in a medium including TGFβ and IL-2 in the presence of anti-CD3 antibody stimulation, a step of culturing the cells in a medium including IL-2 in the absence of anti-CD3 antibody, and a step of culturing the cells again in a medium including TGFβ and IL-2 in the presence of anti-CD3 antibody stimulation.

As used herein, "anti-CD3 antibody stimulation" means that stimulation specific to a CD3 receptor on a cell is provided. Examples of the CD3 stimulation include an anti-CD3 agonistic antibody. The anti-CD3 agonistic antibody may be prepared by using a commercially available product as a research reagent or by a conventional method. As the anti-CD3 antibody, for example, an antibody derived from an animal such as a mouse, a rabbit, a goat, or a cow, and an antibody derived from a human can be used.

In one embodiment, the medium used in each step may further include retinoic acid and/or ascorbic acid. Preferably, the medium can include ascorbic acid. In one embodiment, the medium may further include a CDK8 inhibitor, a CDK19 inhibitor, and/or a CDK8/19 inhibitor. Preferably, the medium can include a CDK8 inhibitor, a CDK19 inhibitor, and/or a CDK8/19 inhibitor.

In one embodiment of the disclosure, the first basal medium and the second basal medium can each independently include at least one, at least two, at least three, at least four, at least five or all factors selected from the group consisting of an anti-CD3 antibody, TGF-β1, IL-2, retinoic acid, a CDK8 inhibitor, a CDK19 inhibitor, a CDK8/19 inhibitor, and ascorbic acid. In still another embodiment, the first basal medium and the second basal medium can each independently include an anti-CD3 antibody, TGF-β1, IL-2, retinoic acid, a CDK8 inhibitor, a CDK19 inhibitor, a CDK8/19 inhibitor, and ascorbic acid. The concentration included as each of these components can be a normal concentration used in the art. In one embodiment, the concentration of the CDK8 inhibitor, the CDK19 inhibitor, and/or the CDK8/19 inhibitor that may be used may be any suitable concentration that may be used in the art, for example, when SenexinA is used, about 0.1µM or more, about 0.5µM or more, about 1µM or more, about 2µM or more, about 3µM or more, about 4µM or more, about 5µM or more, about 6µM or more, about 7µM or more, about 8µM or more, about 9µM or more, about 10µM or more, about 12µM or more, about 14µM or more, about 16µM or more, about 18µM or more, about 20µM or more, and the like, but is not limited to these concentrations, and can be appropriately changed by a person skilled in the art according to other medium compositions.

In one embodiment, in the present disclosure, regulatory T cells are produced from human peripheral T cells. The peripheral T cells include naive regulatory T cells, CD4-positive T cells, CD8-positive T cells, and the like. The regulatory T cells may be induced from a culture including a plurality of types of T cells, or the regulatory T cells may be induced after specific cells such as CD4-positive T cells and CD8-positive T cells are isolated from these cells. In addition, regulatory T cells may be induced after T cells specific to a specific antigen are isolated. Thus, inducible regulatory T cells of the disclosure include those that are CD4 positive, CD8 positive. As used herein, the term "CD4 positive" or "CD4+" refers to a CD4-positive CD8-negative single positive cell unless otherwise specified. In addition, in the production method in the present disclosure, either CD4-positive or CD8-positive T cells can be used as a raw material, and even after generation of inducible regulatory T cells, the CD4-positive or CD8-positive characteristics can be maintained unless special manipulation is performed. The inducible regulatory T cells containing a chimeric antigen receptor (CAR) in the present disclosure are also preferred because similar properties are maintained.

In one embodiment, in the method of the present disclosure, the antibody may be added to the culture medium, or may be immobilized on the inner wall of the culture vessel or the surface of the insoluble carrier. As the insoluble carrier, a member capable of physically or chemically binding the anti-CD3 antibody and insoluble in an aqueous solution can be used. Examples of the material capable of physically adsorbing the anti-CD3 antibody include synthetic resins such as polystyrene, polyethylene terephthalate, polycarbonate, and polypropylene, and glass. The shape of the insoluble carrier is not particularly limited, and for example, a plate shape, a bead shape, a container shape, or the like can be adopted. The amount of the anti-CD3 antibody varies depending on the titer and derivation of the antibody to be used, but may be appropriately set so as to provide sufficient stimulation for induction of regulatory T cells.

In one embodiment, in the method of the present disclosure, a medium obtained by adding a necessary factor to a basal medium for culturing animal cells can be used for culturing cells. Examples of the basal medium for culturing animal cells that can be used in the method of the present disclosure include Iscove's modified Eagle's Medium medium, Ham's F12 medium, MEM Zinc Option medium, IMEM Zinc Option medium, IMDM medium, Medium 199 medium, Eagle's Minimum Essential Medium (EMEM) medium, αMEM medium, Dulbecco's modified Eagle's Medium (DMEM) medium, RPMI 1640 medium, Fischer's medium, and a medium in which a mixed medium or a part thereof is modified.

The basal medium may contain serum (For example, fetal bovine serum (FBS)) or may be serum-free. The serum-free medium may optionally include one or more serum alternatives such as, for example, albumin, bovine serum albumin (BSA), transferrin, apotransferrin, KnockOut Serum Replacement (KSR) (a serum alternative in ES cell culture) (Thermo Fisher Scientific), N2 supplement (Thermo Fisher Scientific), B27 supplement (Thermo Fisher Scientific), fatty acid, insulin, collagen precursor, trace elements, 2-mercaptoethanol, 3'-thiol glycerol, monothioglycerol, and the like. The basal medium may also contain one or more substances, such as a lipid (For example, chemically defined lipid concentrate), an amino acid, L-glutamine, GlutaMAX (Thermo Fisher Scientific), a non-essential amino acid (NEAA), a vitamin (For example, nicotinamide, ascorbic acid), a growth factor, an antibiotic (For example, penicillin and streptomycin), an antioxidant, a pyruvate, buffer, an inorganic salt, and an equivalent thereof.

In one embodiment, the basal medium is RPMI 1640 medium including serum and HEPES(4-(2-hydroxyethyl)-1-piperazineethanesulfonic acid).

In the method of the present disclosure, the cells may be cultured under general animal cell culture conditions. The culture temperature is, but is not limited to, about 30 to 40°C, preferably about 37°C. The culture is preferably carried out in an atmosphere of CO₂-containing air, and the CO₂ concentration is preferably about 2 to 5%.

In the method of the present disclosure, the anti-CD3 antibody may be directly added to the culture medium, or may be used an antibody that is immobilized on the inner wall of the culture vessel or the surface of the insoluble carrier. The amount of the anti-CD3 antibody varies depending on the titer and derivation of the antibody to be used, but may be appropriately set so as to provide sufficient stimulation for induction of regulatory T cells.

TGFβ used includes TGFβ1, TGFβ2, and TGFβ3, for example, TGFβ1. The concentration of TGFβ may be appropriately determined by those skilled in the art, and is not particularly limited. When TGFβ1 or TGFβ3 is used as TGFβ, the concentration in the medium is not particularly limited, but may be 0.25 to 25 ng/mL, for example, about 10 ng/mL.

The concentration of IL-2 in the medium used is not limited, and can be from about 5 U/mL to about 500 U/mL, such as about 100 U/mL.

Retinoic acid and/or ascorbic acid may be further added to the medium used in the present disclosure. Preferably, the medium includes ascorbic acid. The concentration of ascorbic acid is not limited, but is from about 1 to about 100 µg/mL, such as about 10 µg/mL.

To the medium used in the present disclosure, a CDK8 inhibitor, a CDK19 inhibitor, and/or a CDK8/19 inhibitor may be further added. As the CDK8 inhibitor, the CDK19 inhibitor, and/or the CDK8/19 inhibitor, any can be used, and examples thereof include 4-[1-(2-methyl-1H-benzimidazol-5-yl)-1H-imidazo[4,5-c]pyridin-2-yl]-1,2,5-oxadiazol-3-amine, 3-{1-[1-(4-methoxyphenyl)piperidin-4-yl]-4-methyl-1H-imidazo[4,5-c]pyridin-2-yl}pyrazin-2-amine or salts, hydrates, solvates, and the like thereof, or compounds described in U.S. Patent No. 8598344, WO2013/001310, WO2013/040153, WO2013/116786, WO2014/029726, WO2014/063778, WO2014/072435, WO2014/090692, WO2014/106606, WO2014/123900, WO2014/154723, WO2014/194245, WO2015/049325, WO2015/100420, WO2015/144290, WO2015/159937, WO2015/159938, WO2016/009076, or WO2018/139660, and the like. As one example, SenexinA or AS2863619 is exemplified, but the present disclosure is not limited thereto. The concentration of the CDK8 inhibitor, the CDK19 inhibitor, and/or the CDK8/19 inhibitor that may be used may be any suitable concentration that may be used in the art, and may be any suitable concentration described in the above literature, and appropriately can be changed according to other medium compositions by a person skilled in the art.

In the method of the present disclosure, human peripheral T cells are stimulated with an anti-CD3 antibody in a medium including TGFβ and IL-2 to induce regulatory T cells, and after performing dormant culture in a medium containing IL-2 not including an anti-CD3 antibody, human peripheral T cells are stimulated with an anti-CD3 antibody again, whereby regulatory T cells having a high immunosuppressive function can be induced. It can be confirmed that the obtained inducible regulatory T cells have a high immunosuppressive function, for example, by comprehensive gene expression analysis by an RNA sequencing method and a cell proliferation suppression test in vitro.

In one embodiment of the present disclosure, the number of culture days in the step (a) of stimulating CD4-positive T cells or CD8-positive T cells in peripheral blood with the first basal medium for about 1 to about 5 days can be appropriately set by those skilled in the art, and is not particularly limited, but can be, for example, about 3 days.

In one embodiment, the number of days of culture in the dormant culture step in the step (b) can be appropriately set by those skilled in the art, and is not particularly limited, but may be, for example, about 2 days.

In one embodiment, the number of days of culture in the culture step in the second basal medium in the (c) can be appropriately set by those skilled in the art, and is not particularly limited, but may be, for example, about 3 days.

In one embodiment, the number of days of culture in the dormant culture step of step (d) can be appropriately set by those skilled in the art, and is not particularly limited, but may be, for example, about 2 days. In one embodiment, the regulatory T cells having an induced regulatory T cell specific demethylation state can be expanded by culturing in an unstimulated state in the presence of IL-2. A stable regulatory T cell culture of regulatory T cells induced by the fact that the medium may further include ascorbic acid and by culturing in a medium further including IL-2 can be obtained.

In order to isolate the regulatory T cells from the obtained cell culture including the regulatory T cells, the cells may be isolated by a conventional method based on cell surface markers unique to the regulatory T cells, and for example, the FoxP3-positive fraction may be taken out by a cell sorter. Regulatory T cells having specific antigenic properties may also be isolated as desired.

The inducible regulatory T cells obtained by the method of the present invention are expected to be used for the treatment of human inflammatory diseases, for example, an autoimmune disease and an allergy.

### (Measurement of positive rate by flow cytometry)

In one embodiment, the positive rate measurement by flow cytometry can be performed as follows.

### Preparation

· Fixation/Permeabilization Concentrate (Hereinafter, Buffer) (eBio Science, 00-5123-43) · Fixation/Permeabilization Diluent (Hereinafter, Diluent) (eBio Science, 00-5223-56) · Permeabilization Buffer (10×) (eBio Science, 00-833-56)
· FOXP3 Monoclonal Antibody (236A/E7), PE (Hereinafter, anti-FOXP3 antibody) (eBio Science, 12-4777-42)
· Mouse IgG1 kappa Isotype Control (P3.6.2.8.1), PE (PE control) (eBio Science) · BV421, Mouse, Anti-Human, CD152 (Hereinafter, an anti-CTLA4 antibody) (BD) · BV421 Mouse IgG2a, k Isotype Control (BV421 control) (BD)
· CD4 Monoclonal Antibody (RPA-T4), APC (Hereinafter, anti-CD4 antibodies) (eBio Science)
· Mouse IgG1 kappa Isotype Control (P3.6.2.8.1), APC (Hereinafter, APC control) (eBio Science)
· D-PBS (NACALAI TESQUE, INC., 14249-95)
· FBS (HyClone, SH30084.03)
· 0.5 mol/l-EDTA solution (pH 8.0) (NACALAI TESQUE, INC., 06894-14) · MilliQ water
· Centrifuge
· Safety cabinet
· Micropipette (P200, P1000)
· 5 mL polystyrene round tube (Hereinafter, the dedicated tube) (Falcon, 352008) · Nylon mesh (65 µm) (Kyoshin Rikou, PP-65N)

### Reagent preparation

*Fixation Buffer (100 µL used per sample)
Buffer and Diluent are mixed at a ratio of 1 : 3.
*Perm Buffer
Permeabilization Buffer (10 x) is diluted 10 folds with MilliQ water.
*FACS Bufer (for 500 mL preparation)

| | |
|---|---|
| D-PBS | 489 mL |
| FBS | 10 mL (final concentration 2%) |
| 0.5 mol/l EDTA solution | 1 mL (final concentration: 1 mM) |

The above reagents are stored at 4°C or on ice after preparation.

### Method

(1) Add 500 µL of FACS Bufer to a 1.5 mL tube.
(2) Add 1 × 10⁶ end products to the tube of (1) and gently suspend with a micropipette.
(3) Centrifuge 500 ×g at 4°C for 5 minutes in a centrifuge.
(4) After removing the supernatant with an aspirator, add 100 µL of Fixation Buffer and gently pipette it. -> Be careful not to whip.
(5) Fix the sample by standing for 30 minutes or more with light shielding on ice. -> 45 minutes is acceptable.
(6) After fixation, add 1 mL of Perm Buffer to the tube and gently pipette it.
(7) Prepare a new 1.5 mL tube for the number of samples.
(8) Dispense 500 µL of sample each for control and antibody staining.
(9) Centrifuge 500 ×g at 4°C for 5 minutes in a centrifuge.
(10) An anti-FOXP3 antibody (stock solution concentration = 0.05 mg/ml), an anti-CTLA4 antibody (Lot: 0030269 stock solution concentration = 0.2 mg/ml), and an anti-CD4 antibody (stock solution concentration = 0.1 mg/ml) are prepared in a 100 fold dilution with Perm Buffer during centrifugation (Hereinafter, an antibody preparation solution). For control samples, PE control (stock solution concentration = 0.1 mg/ml), BV421 control (stock solution concentration = 0.2 mg/ml) and APC control (stock solution concentration = 0.1 mg/ml) are adjusted to the same concentration as the antibody of the corresponding dye (hereinafter referred to as control solution).
   *CTLA4 staining is not performed in the in-step control test.
(11) After removing the supernatant with an aspirator, add 100 µL each of an antibody preparation solution to the antibody-stained sample and a control solution to the control sample, and gently pipette it. -> Be careful not to whip.
(12) Fix the sample by standing for 60 minutes with light shielding on ice.
(13) Perform start-up of measuring instrument during staining.
(14) After staining, add 1 mL of FACS Bufer and gently pipette it.
(15) Centrifuge 500 ×g at 4°C for 5 minutes in a centrifuge. ->After removing the supernatant with an aspirator, wash (14) and (15) again repeatedly.
(16) After removing the supernatant with an aspirator, add 500 µL of FACS Bufer and gently pipette it.
(17) Place a nylon mesh on the dedicated tube with tweezers, and filter the suspension.
(18) Analyze it with any flow cytometer instrument. The number of data collected cells is 10,000 or more. In calculation of the target antigen positive rate, a reference line is drawn so that the positive rate of the control sample is 5% or less, and the proportion of cells showing an antigen expression level equal to or higher than the reference is taken as the positive rate.

### Remarks

· Reagents used for fixation and staining can be purchased in sets of three as "Foxp3/Transcription Factor Staining Buffer Set" (Cat.: 00-5523).
· There is no problem with the device settings and the like as long as they do not deviate to the extent that it can be clearly determined from a general and scientific viewpoint that normal measurement has not been performed. The clear deviation is a case where various signals of the cell population of interest fall below a set fluorescence threshold value, a case where various signal values of a control sample or a sample to be measured fall below or exceed a limit value that can be normally measured by a device, or the like.

### (Measurement of immunosuppressive activity)

The cells of the present disclosure may have immunosuppressive activity. The immunosuppressive activity can be measured by various methods.

In one embodiment, as described in the Examples, the suppression can be determined by measuring cell proliferation due to an immune reaction caused by responder T cells. Preferably, the present disclosure desirably demonstrates using in vivo models. For example, as described in Examples, in the case of the colitis model mouse, a tissue is collected after a certain period of administration from the colitis model mouse to which the inducible regulatory T cells of the present disclosure are administered, and its activity can be measured depending on whether or not immunosuppression is confirmed in the tissue. Confirmation of immunosuppression in tissues can be achieved by various methods, and for example, the presence or absence of immunosuppression can be confirmed by staining analysis of tissues.

### <Chimeric antigen receptor (CAR)>

In an aspect of the present disclosure, there are provided the inducible regulatory T cells containing a chimeric antigen receptor (CAR). The CAR contained in the inducible regulatory T cells of the present disclosure may be contained as a protein as long as it can function as a CAR, or may include a nucleic acid molecule expressing the CAR. In one embodiment, the CAR of the present disclosure can be expressed in inducible regulatory T cells.

The CAR disclosed herein includes at least one extracellular domain, at least one transmembrane domain, and at least one intracellular domain capable of binding an antigen.

A chimeric antigen receptor (CAR) is an artificially constructed hybrid protein or polypeptide that contains an antigen-binding domain (For example, a single chain variable fragment (scFv)) of an antibody linked via a transmembrane domain to a T cell signaling domain. CAR characteristics include redirecting T cell specificity and reactivity towards a selected target, regardless of MHC, exploiting the antigen-binding properties of a monoclonal antibody. Non-MHC antigen recognition can give T cells expressing the CAR the capacity to recognize an antigen independently of antigen processing.

The intracellular T-cell signaling domain of the CAR can include, for example, a T-cell receptor signaling domain, a T-cell co-stimulatory signaling domain, or both. The T-cell receptor signaling domain refers to a part of the CAR that includes an intracellular domain of a T cell receptor, such as an intracellular part of a CD3 zeta protein. A co-stimulatory signaling domain refers to a part of the CAR that includes an intracellular domain of a co-stimulatory molecule that is a cell surface molecule other than an antigen receptor or their ligands that is required for an efficient response of lymphocytes to an antigen.

### (Extracellular domain)

In one embodiment, the CAR used in the inducible regulatory T cells disclosed herein includes an antigen-binding domain or part thereof. The antigen-binding domain or part thereof can be selected as appropriate depending on the type and number of ligands on the surface of the target cell, for example, the antigen-binding domain can be selected to recognize a ligand that acts as a cell surface marker on the target cell associated with a particular disease state. Thus, examples of the cell surface marker that may act as a ligand for the antigen-binding domain in the CAR of the present disclosure can include those associated with a tissue-specific marker, a cytoma-specific marker, viral, bacterial and parasitic infections, an autoimmune disease, and cancer cells.

In one embodiment, the antigen-binding domain part of the CAR of the present disclosure can target an antigen, including targeting molecules including (1) an alloantigen, including MHC class I, MHC class II, and the like; (2) an extracellular autoantigen including TSHR (thyroid stimulating hormone receptor), DSG3 (desmoglein 3), Cytokeratin8, and the like; (3) a foreign antigen including Gliadin, Ara h2, etc.; (4) an antigen including targeting molecules including CD4, CD8, CD19, BCMA, CD68, MSLN (mesothelin), MadCam1(mucosal vascular addressin cell adhesion molecule 1), and the like, but an antigen to which the antigen-binding domain part of the CAR of the present disclosure may be targeted are not limited thereto.

In one embodiment, depending on the desired antigen to be targeted, the CAR of the present disclosure can be modified to include an antigen binding domain specific to the desired antigen target. For example, if CD19 is the target antigen, an antibody against CD19 may be used as the antigen binding domain into the CAR.

### (Transmembrane domain)

The CAR used in the inducible regulatory T cells disclosed herein can include one or more transmembrane domains fused to the extracellular domain.

In one embodiment, a linker domain derived from the extracellular domain may be linked to the transmembrane domain. The transmembrane domain may be natural or synthetic and may be derived from any membrane-bound or transmembrane protein as a natural transmembrane domain. Transmembrane regions particularly used in the present disclosure may be derived from the alpha, beta or zeta chain of the T cell receptor, CD28, CD3 epsilon, CD45, CD4, CD5, CD8, CD9, CD16, CD22, CD33, CD37, CD64, CD80, CD86, CD134, CD137, CD154, CD271, TNFRSF19, and the like.

In one embodiment, the CAR used in the inducible regulatory T cells disclosed herein can also place a spacer domain between the extracellular domain and the transmembrane domain or between the intracellular domain and the transmembrane domain. The spacer domain preferably can have a sequence that promotes binding of the CAR to the antigen and enhances signaling into the cell.

### (Intracellular domain)

The cytoplasmic signaling domain (or intracellular signaling domain) of the CAR is responsible for activating at least one of the normal effector functions of the immune cell in which the CAR is expressed. The "effector function" means a specialized function of a cell, for example, in a Treg, the effector function can be a suppressive or regulatory activity, including secretion of immunosuppressive cytokines such as IL-10, IL-35, TGF-β, or expression of inhibitory molecules such as TIGIT, CTLA4, competitive cytokine receptors such as the IL-2 receptor. An intracellular signaling domain refers to a part of a protein that transduces the effector function signal and directs the cell expressing the CAR to perform a specialized function. The intracellular signaling domain may include any complete, mutant, or truncated part of the intracellular signaling domain of a predetermined protein sufficient to transmit a signal that initiates or blocks an immune cell effector function.

In one embodiment, examples of intracellular signaling domains employed in the CAR include the cytoplasmic signaling sequences of the T cell receptor (TCR) and a coreceptor that initiate signaling following antigen receptor binding.

### (Alloantigen, allergen and hapten associated with rejection)

The CAR used in inducible regulatory T cells disclosed herein can include those associated with an alloantigen, an allergen and a hapten associated with rejection.

### <Production of inducible regulatory T cells containing chimeric antigen receptor (CAR) >

In an aspect of the present disclosure, there is provided a method of producing inducible regulatory T cells containing a chimeric antigen receptor (CAR), the method including: (a) a step of stimulating CD4-positive T cells or CD8-positive T cells in peripheral blood with a first basal medium for about 1 to about 5 days; (b) a step of dormant culturing cells obtained in step (a) in a medium including IL-2 for at least about 1 to about 3 days; (c) a step of stimulating cells obtained in step (b) with a second basal medium for about 1 to about 5 days; (d) a step of dormant culturing cells obtained in step (c) in a medium including IL-2 for at least about 1 to about 3 days; and (e) a step of introducing the CAR gene at least once in any of steps (a) to (d). In one embodiment, the method for producing inducible regulatory T cells containing a chimeric antigen receptor (CAR) of the present disclosure can be performed by introducing a CAR gene when producing the inducible regulatory T cells of the present disclosure.

In one embodiment, the introduction of the CAR gene can be performed at least twice in total in any of the above steps (a) to (d), and in this way, the CAR can be stably expressed. In one embodiment, the introduction of the CAR gene can be performed at least once in the step (a).

In one embodiment of the disclosure, the method of producing inducible regulatory T cells containing a chimeric antigen receptor (CAR) can include one or more characteristics of the method of producing inducible regulatory T cells.

### <Pharmaceutical use of inducible regulatory T cells containing chimeric antigen receptor (CAR)>

In an aspect of the disclosure, there is provided a pharmaceutical composition including inducible regulatory T cells containing a chimeric antigen receptor (CAR). In one embodiment of the disclosure, the inducible regulatory T cells in the disclosure can have one or more characteristics of the inducible regulatory T cells described above.

Also provided in one aspect is a therapeutic agent for a T cell associated disease including inducible regulatory T cells or a cell population of the disclosure, wherein a diagnosis is made with respect to the T cell-associated disease in a diseased subject, and an appropriate CAR contained in the inducible regulatory T cells or the cell population is selected based on the diagnosis.

In another aspect of the present disclosure, there is provided a method of treating or preventing a disease, disorder or condition that can be treated with the inducible regulatory T cells in a subject, including a step of administering to the subject an effective amount of the inducible regulatory T cells containing a chimeric antigen receptor (CAR) or a cell population including the same. In one embodiment of the disclosure, the inducible regulatory T cells in the disclosure can have one or more characteristics of the inducible regulatory T cells described above.

The methods of the present disclosure can be directed to treating or preventing any disease, disorder, or condition that can be treated with inducible regulatory T cells. Generally, any disease, disorder or condition in which regulatory T cells are effective even a little can be directed, but is not limited thereto, and it is understood that inducible regulatory T cells are also encompassed as they may be efficacious even when normal regulatory T cells are not efficacious.

Pharmaceutical compositions including inducible regulatory T cells containing a chimeric antigen receptor (CAR) of the disclosure can be used as a pharmaceutical composition for the treatment or prevention of an autoimmune disease, an allergy, graft rejection, chronic inflammatory diseases such as a graft versus host disease, inflammatory bowel disease, or chronic infections by viruses, bacteria, parasites in a subject.

In one embodiment, a pharmaceutical composition including inducible regulatory T cells containing a chimeric antigen receptor (CAR) of the disclosure can be used for cell therapy. In the cell therapy, inducible regulatory T cells containing a chimeric antigen receptor (CAR) of the disclosure can be infused into a subject in need thereof as a pharmaceutical composition or as a formulation of a therapeutically effective cell population expressing a CAR of the disclosure. Infused Treg cells in a subject may be able to suppress the subject's inflammatory immune response or at least reduce the effect and/or condition of the disorder being treated. The subject may be the same subject from which the cells were obtained (autologous cell therapy) or the cells may be derived from another subject of the same species (allogeneic cell therapy).

In one embodiment, a Treg cell or population thereof containing a CAR of the present disclosure can be formulated for administration to a subject using techniques publicly known to those skilled in the art. In one embodiment, a formulation including a therapeutically effective Treg cell or population thereof containing a CAR of the present disclosure may include a pharmaceutically acceptable excipient (carrier or diluent). The excipients included in the formulations have different purposes, for example, depending on the nature of the antigen-binding domain of the CAR of the present disclosure. Examples of commonly used excipients include, but are not limited to: saline, buffered saline, dextrose, water for injection, glycerol, ethanol, and combinations thereof, a stabilizer, a solubilizer and a surfactant, a buffer and a preservative, an isotonizing agent, a bulking agent, and a lubricant.

Formulations including therapeutically effective Treg cells or a population thereof containing the CAR of the present disclosure may be administered to a subject using manners and techniques publicly known to those skilled in the art. Exemplary include, but are not limited to, intravenous injection. Other modalities include, but are not limited to, intratumoral, intradermal, subcutaneous, intramuscular, intraperitoneal, intraarterial, intramedullary, intracardiac, intraarticular (joint), intrasynovial (joint fluid area), intracranial, intraspinal, and intrathecal (cerebrospinal fluid), and the like.

### (General technology)

Molecular, biochemical, and microbiological techniques used herein are well known and commonly used in the art, e.g., Sambrook J. et al. (1989). Molecular Cloning: A Laboratory Manual, Cold Spring Harbor, and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, and extra issue Experimental Medicine "Gene Transfer & Expression Analysis Experimental Method" YODOSHA CO., LTD., 1997, etc., relevant parts (which may be all) of which are incorporated herein by reference.

For DNA synthesis techniques and nucleic acid chemistry for producing artificially synthesized genes, gene synthesis and fragment synthesis services such as GeneArt, GenScript, and Integrated DNA Technologies (IDT) can also be used, and others are described in for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; and Blackburn, G.M. et al. (1996). Nucleic Acids in Chemistry and Biology, Oxford University Press; Hermanson, G.T. (I996). Bioconjugate Techniques, Academic Press, etc., and the relevant parts of these are incorporated herein by reference.

"Or" is used herein when "at least one or more" of the items listed in the text can be employed. The same applies to "or". When it is stated herein that "within the range" of "two values", the range includes the two values themselves.

References cited herein, such as scientific literature, patents, patent applications, and the like, are incorporated by reference herein in their entireties to the same extent as each was specifically described.

The present disclosure has been described above with reference to preferred embodiments for easy understanding. Hereinafter, the present disclosure is described based on examples, but the above description and the following examples are provided for the purpose of illustration only and not for the purpose of limiting the present disclosure. Accordingly, the scope of the present disclosure is not limited to the embodiments or examples specifically described herein, but is limited only by the claims.

### Examples

In the following examples, glutamine-free RPMI1640 (10% FCS (v/v), 60 µg/mL penicillin G, 100 µg/mL streptomycin, including 10 mM HEPES) was used as a basal medium. If necessary, 30 to 300 mg/L of L-glutamine was added to the medium and used.

### Bisulfite sequence

Genomic DNA was obtained from the induced cells, and after treatment using MethylEasy Xceed Rapid DNA Bisulphite Modification Kit (Human Genetic Signatures), the demethylation of genes characteristic of regulatory T cells was examined. Analysis of demethylation of each gene used known methods and primers (Floess et al. (2007) PloS Biology Volume 5, Issue 2, e38, and Ohkura et al. (2012) Immunity 37 (5) 785-799).

In the analysis of the human Foxp3 CNS2 region, 5'-TTGGGTTAAGTTTGTTGTAGGATAG-3' (SEQ ID NO: 1) was used on the forward side, and 5'-ATCTAAACCCTATTATCACAACCCC-3' (SEQ ID NO: 2) was used on the reverse side.

### Obtainment of fraction of mouse T cells

Foxp3-eGFP (eFox) reporter mice (Ito et al. (2014) Science 346, 363-368) were used. Lymph node cells of Foxp3-eGFP (eFox) reporter mice were obtained and an endogenous regulatory T cell (nTreg) fraction was obtained by sorting CD4⁺GFP⁺ cells by FACSAriaII (BD). CD4⁺GFP⁻CD44^{low}CD62L^{high} cells were sorted to obtain a naive T cell fraction. Furthermore, a CD4⁺GFP⁻CD44^{high}CD62L^{low} cell fraction was obtained and used as effector/memory T cells.

### Obtainment of fraction of human T cells

Human CD4⁺ T cells were isolated from PBMCs of a Crohn's disease patient. The concentrated PBMCs were purified using CliniMACS CD4 GMP MicroBeads according to the product protocol.

### (Example 1: Method for producing mouse highly functional stable regulatory T cells)

Mouse CD4-positive T cells were stimulated with basal medium including anti-CD3 antibody (the antibody having a concentration of 10 µg/ml is added in an amount of 100 µL per well, and the plate is allowed to stand at room temperature for 60 minutes to immobilize it on the container), hIL-2 (100 U/ml), hTGFβ1 (2.5 ng/ml), retinoic acid (1µM), SenexinA (5µM) for 3 days. Then, the cells were subjected to dormant culture in a basal medium including hIL-2 (100 U/ml) for 2 days. After 2 days, the medium was replaced in the same manner, and dormant culture was further performed for 2 days. After 2 days, the cells were stimulated again for 2 days with a basal medium including an anti-CD3 antibody (the antibody having a concentration of 10 µg/ml is added in an amount of 100 µL per well, and the plate is allowed to stand at room temperature for 60 minutes to immobilize it on the container), hIL-2 (100 U/ml), hTGFβ1 (2.5 ng/ml), retinoic acid (1µM), SenexinA (5µM), and ascorbic acid (10 µg/ml). Then, the cells were subjected to dormant culture in a basal medium including hIL-2 (100 U/ml) for 2 days. After 2 days, the medium was replaced in the same manner, and dormant culture was further performed for 2 days. The expression of FoxP3 and CD25 in the regulatory T cells obtained after 2 days was analyzed by a flow cytometry method (FIG. 1), the demethylation state of the Treg-specific demethylated region was analyzed by a bisulfite method (FIG. 2), and the global gene expression pattern was analyzed by an RNA-seq method (FIG. 3).

Although conventional inducible regulatory T cells (conventional iTreg) have insufficient expression of a functional molecule (in this case, for example, CTLA4, Entpd1, Nt5e, Itgae, and the like are illustrated in FIG. 3) and low FoxP3 induction efficiency, and it could be confirmed that the highly functional inducible regulatory T cells obtained by the preparation method of the present application highly express FoxP3 and functional genes (in this case, for example, CTLA4, Entpd1, Nt5e, Itgae, and the like are illustrated in FIG. 3) and have a demethylation state characteristic of Treg (FIG. 1 to 3).

### (Example 2: In vitro suppression activity of regulatory T cells)

Regulatory T cells were obtained by the same experimental system as in Example 1. The responder T cells (5 × 10⁴) labeled with Cell Trace Violet and the produced regulatory T cells (5 × 10⁴) were mixed and cultured for 3 days in the presence of anti-CD3 antibody (1 µg/ml) + antigen-presenting cells (MHC-II positive cells: 1 × 10⁴) or Dynabeads T-activator (Veritas Corporation) (5 µL/well), and the Cell Trace Violet intensity was analyzed by flow cytometry method. When responder T cells initiate an immune reaction, the Cell Trace Violet intensity shows a plurality of weak peaks due to cell proliferation, but when this immune reaction is suppressed, the Cell Trace Violet intensity is retained as a single strong peak. It was confirmed that the regulatory T cells obtained by the preparation method of the present application strongly suppressed the immune reaction of the responder T cells. Therefore, it was confirmed that the obtained regulatory T cells have a higher suppressive function than the existing regulatory T cell population (FIG. 4).

### (Example 3: In vivo stability of regulatory T cells)

Regulatory T cells were produced from Thy1.2/eFox reporter mice by the method of Example 1, Foxp3 positive cells (2 × 10⁵) were separated by FACS, and Thy1.1/wild type mice were tail-vein administered. Thy1.2 cells transferred after 2 weeks were collected from the lymph node, and Foxp3 expression was analyzed (FIG. 5). It was confirmed that the regulatory T cells induced without CD28 stimulation and subjected to stabilized culture stably expressed Foxp3 even after 2 weeks in vivo.

### (Example 4: suppression effect on colitis model)

2 × 10⁵ wild type mouse-derived CD45 RB-highly expressing CD4 positive naive T cells were administered to a RAG-deficient mouse to produce a colitis model. After 1 week, 2 × 10⁵ highly functional stable iTreg cells produced by the method of Example 1 were administered, and the body weight change was measured over time (FIG. 6A). Thereafter, 1 month after the administration, large intestine tissues and lymph nodes were collected, and HE staining analysis of the large intestine tissues (FIG. 6B) and CD69 expression analysis in lymph node T cells (FIG. 6C: flow cytometry method) were performed. As a result, it was confirmed that the administration of the highly functional stable iTreg suppressed colitis. In addition, the treatment effect was confirmed at a dose of 2 × 10⁵ cells per mouse (about 20 g), and the efficacy at 1 × 10⁷ cells/kg was confirmed.

### (Example 5: Production of regulatory T cells from human peripheral T cells)

CD4-positive T cells derived from a Crohn's disease patient were stimulated with basal medium including anti-CD3 antibody (the antibody having a concentration of 10 µg/ml is added in an amount of 100 µL per well, and the plate is allowed to stand at room temperature for 60 minutes to immobilize it on the container), hIL-2 (100 U/ml), hTGFβ1 (10 ng/ml), retinoic acid (1µM), SenexinA (5µM) for 3 days. Then, the cells were subjected to dormant culture in a basal medium including hIL-2 (100 U/ml) for 2 days. After 2 days, the medium was replaced in the same manner, and dormant culture was further performed for 2 days. After 2 days, the cells were stimulated again for 2 days with a basal medium including an anti-CD3 antibody (the antibody having a concentration of 10 µg/ml is added in an amount of 100 µL per well, and the plate is allowed to stand at room temperature for 60 minutes to immobilize it on the container), hIL-2 (100 U/ml), hTGFβ1 (10 ng/ml), retinoic acid (1µM), SenexinA (5µM), and ascorbic acid (10 µg/ml). Then, the cells were subjected to dormant culture in a basal medium including hIL-2 (100 U/ml) for 2 days. After 2 days, the medium was replaced in the same manner, and dormant culture was further performed for 2 days. The expression of FoxP3 and CTLA4 of the regulatory T cells obtained after 2 days was analyzed by flow cytometry (FIG. 7A).

Although conventional inducible regulatory T cells (conventional iTreg) have insufficient expression of a functional molecule (in this case, CTLA4) and low FoxP3 induction efficiency, it can be confirmed that the highly functional inducible regulatory T cells obtained by the preparation method of the present application are a population including many cells expressing FoxP3 and CTLA4.

### (Example 6: In vitro suppressive activity of regulatory T cells)

Regulatory T cells were obtained by the same experimental system as in Example 1. The responder T cells (5 × 10⁴) labeled with Cell Trace Violet and the produced regulatory T cells (5 × 10⁴) were mixed and cultured for 3 days in the presence of Treg Suppression Inspector (Miltenyi Biotec) (5 µL/well), and the Cell Trace Violet intensity was analyzed by flow cytometry method. When responder T cells initiate an immune reaction, the Cell Trace Violet intensity shows a plurality of weak peaks due to cell proliferation, but when this immune reaction is suppressed, the Cell Trace Violet intensity is retained as a single strong peak. It was confirmed that the regulatory T cells obtained by the preparation method of the present application strongly suppressed the immune reaction of the responder T cells. Therefore, it was confirmed that the obtained regulatory T cells have a higher suppressive function than the existing regulatory T cell population.

### (Example 7: Production of regulatory T cells from CD8-positive human peripheral T cells)

CD8-positive T cells were stimulated with basal medium including anti-CD3 antibody (the antibody having a concentration of 10 µg/ml is added in an amount of 100 µL per well, and the plate is allowed to stand at room temperature for 60 minutes to immobilize it on the container), hIL-2 (100 U/ml), hTGFβ1 (10 ng/ml), retinoic acid (1µM), SenexinA (5µM) for 3 days. Then, the cells were subjected to dormant culture in a basal medium including hIL-2 (100 U/ml) for 2 days. After 2 days, the medium was replaced in the same manner, and dormant culture was further performed for 2 days. After 2 days, the cells were stimulated again for 2 days with a basal medium including an anti-CD3 antibody (the antibody having a concentration of 10 µg/ml is added in an amount of 100 µL per well, and the plate is allowed to stand at room temperature for 60 minutes to immobilize it on the container), hIL-2 (100 U/ml), hTGFβ1 (10 ng/ml), retinoic acid (1µM), SenexinA (5µM), and ascorbic acid (10 µg/ml). Then, the cells were subjected to dormant culture in a basal medium including hIL-2 (100 U/ml) for 2 days. After 2 days, the medium was replaced in the same manner, and dormant culture was further performed for 2 days. The expression of FoxP3, CTLA4, and Helios in the regulatory T cells obtained after 2 days was analyzed by flow cytometry method (FIG. 8).

Even when inducible regulatory T cells were produced from CD8-positive T cells, they were Helios-negative, and could be confirmed to be a population including many cells expressing FoxP3 and CTLA4.

### (Example 7: Introduction of chimeric antigen receptor into inducible regulatory T cells)

In the example, a process of producing highly functional inducible regulatory T cells (HSF-iTreg) from human CD4-positive T cells and the timing of infecting a lentivirus carrying a CAR expressing viral vector in the process were examined.

### (Materials and methods)

The detailed protocol is shown in FIG. 9 (top). Here, the process of producing highly functional inducible regulatory T cells (HSF-iTreg) from human CD4-positive T cells and the timing of infecting the CAR-expressing viral vector-carrying lentivirus in the process were shown.

In the example, highly functional inducible regulatory T cells (HSF-iTreg) were produced from human CD4-positive T cells. As shown in the schematic diagram at the top of FIG. 9, in the process of production of the HSF-iTreg, a CAR expressing viral vector-carrying lentivirus was infected. At the timing indicated by the arrow in FIG. 9, lentivirus (Lenti-CD19 CAR (scFv-CD28, FMC63) Viral Particle, Cat. No.: VP-CAR-LC61) was added in an amount of 1/20 of the total amount of culture liquid, and the expression of FOXP3 and CAR was analyzed by FCM on the final day.

### (Results)

The results are shown in the bottom of FIG. 9. FCM analysis of FOXP3 and CAR expression on the final day after addition of lentivirus (Lenti-CD19 CAR (scFv-CD28, FMC63) Viral Particle, Cat. No.: VP-CAR-LC61) in an amount of 1/20 of the total amount of culture liquid at the timing indicated by the arrow in FIG. 9 (top) shows FOXP3 and CAR gene expression on Day 13 as shown in FIG. 11 (bottom). It was demonstrated that the CAR can be expressed with high efficiency by introducing the gene during the primary stimulation period. The bottom of FIG. 9 shows the results of FCM analysis on the expression of CTLA4 for the Day1 introduced sample. As shown, expression of FOXP3 and CAR genes was shown on Day 13, and high CTLA4 expression was also confirmed in the HSF-iTreg cell population expressing the CAR. It was possible to highly efficiently express the CAR by introducing the gene during the primary stimulation period.

### (Example 8: Expression analysis of functional molecule in CAR Treg)

In the example, the expression of CTLA4 was analyzed by FCM for the sample introduced on Day 1 of Example 7. As a result, high CTLA4 expression could be confirmed also in the HSF-iTreg cell population expressing the CAR.

As an example, when the expression of the CD103 molecule encoded by ITGAE is analyzed by a flow cytometry method, it can be confirmed that the inducible regulatory T cells obtained by the production method of the present application have higher CD103 expression as compared with other regulatory T cell populations. As a comprehensive consideration, it can be expected that the inducible regulatory T cells obtained by the production method of the present application exhibit a high suppression capacity as compared with existing regulatory T cells.

### In addition, expression of Helios can be analyzed by flow cytometry method.

Conventional inducible regulatory T cells (conventional iTreg) have insufficient expression of a functional molecule (For example, in this case, CTLA4) and low FoxP3 induction efficiency. On the other hand, CTLA4 and FoxP3 are expressed in endogenous regulatory T cells (nTreg), and representative Helios are expressed as nTreg marker molecules. On the other hand, it can be confirmed that highly functional inducible regulatory T cells (CAR Treg) expressing the CAR obtained by the preparation method of the present application are Helios-negative, and a population including a large number of cells expressing FoxP3 and CTLA4.

### (Example 9: Evaluation of antigen specificity of CAR Treg)

CAR expression regulatory T cells generated using CD19 positive cells or hCD19 recombinant protein are stimulated, and FOXP3, CTLA4 expression of the stimulated CAR expression regulatory T cells is analyzed by flow cytometry.

### (Example 10: Evaluation of Immunosuppressive Function of CAR Treg)

The immunosuppressive capacity against CD19 positive cells is evaluated in vitro. The CD19 positive cells and the CAR expression regulatory T cells are co-cultured, and the immunological properties of the CD19 positive cells are evaluated by flow cytometry.

### (Example 11: FCM Analysis of highly functional inducible regulatory T cells (HSF-iTreg) from human CD4-positive T cells)

In the example, FCM analysis of highly functional inducible regulatory T cells (HSF-iTreg) was performed from human CD4-positive T cells.

Specifically, as shown in FIG. 11, highly functional inducible regulatory T cells (HSF-iTreg) were produced from human CD4-positive T cells, and lentivirus (Lenti-HLA-A2 CAR(scFv-28ζ, BB7.2)-VP (VP-CAR-LC809) or Lenti-EpCAM CAR(scFv-28ζ, M13-57)-VP (VP-CAR-LC847)) was added at the timing described in the above example in an amount of 1/50 or 1/25 of the total amount of culture liquid, respectively, and the expression of CAR (GFP), FOXP3 and CTLA4 was analyzed by FCM on the final day.

### (Results)

As shown in FIG. 11, it was shown that any type of CAR can be highly efficiently expressed without impairing the properties and induction efficiency of the Treg of the present disclosure by introducing a gene during the primary stimulation period.

### (Note)

Although the present disclosure has been illustrated using preferred embodiments of the present disclosure as above, it is understood that the scope of the present disclosure should be interpreted only by the claims. It is understood that the patents, patent applications, and other documents cited herein are to be incorporated by reference herein in the same manner as their content is specifically described herein. This application claims priority to Japanese Patent Application No. 2022-152882 filed on September 26, 2022 in Japan, which is incorporated herein by reference in its entirety.

### Industrial Applicability

The cell population of the present disclosure can be used for treatment and prevention of various immune diseases and inflammatory diseases such as autoimmune diseases. In addition, it is possible to stably induce regulatory T cells having high functionality from peripheral T cells by the method for producing a cell population of the present disclosure, and application to the medical field can be expected.

### [Sequence Listing Free Text]

SEQ ID NO: 1: Forward primer used in example
SEQ ID NO: 2: Reverse primer used in example

## Claims

1. Inducible regulatory T cells containing a chimeric antigen receptor (CAR).

2. The inducible regulatory T cells of claim 1, wherein the CAR is expressed in the inducible regulatory T cells.

3. The inducible regulatory T cells according to claim 1 or 2, having at least one characteristic selected from the group consisting of CTLA4 positive, NT5E positive, ITGAE (CD103) positive, and AREG positive.

4. The inducible regulatory T cells of any one of claims 1 to 3, having at least two characteristics selected from the group consisting of CTLA4 positive, NT5E positive, ITGAE (CD103) positive, and AREG positive.

5. The inducible regulatory T cells of any one of claims 1 to 4, which are at least CTLA4 positive.

6. The inducible regulatory T cells of any one of claims 1 to 5, wherein the CNS2 site of the FOXP3 gene is demethylated.

7. The inducible regulatory T cells of any one of claims 1 to 6, which are CD4 positive or CD8 positive.

8. The inducible regulatory T cells of any one of claims 1 to 7, obtained from or induced from a human peripheral blood T cell, or a human tissue-derived T cell.

9. The inducible regulatory T cells of any one of claims 1 to 8, wherein the inducible regulatory T cells are obtained by a method including:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

10. A cell population comprising T cells, wherein about 50% or more of the T cells of the cell population are inducible regulatory T cells of any one of claim 1 to 9.

11. The cell population of claim 10, wherein about 80% or more of the T cells in the cell population are inducible regulatory T cells of any one of claim 1 to 9.

12. The cell population of claim 10 or 11, wherein the T cells in the cell population are regulatory T cells.

13. The cell population of any one of claims 10 to 12, wherein about 90% or more of the cell population is T cells.

14. A pharmaceutical composition comprising inducible regulatory T cells containing a chimeric antigen receptor (CAR).

15. The pharmaceutical composition of claim 14, wherein the CAR is expressed on the inducible regulatory T cell.

16. The pharmaceutical composition of claim 14 or 15, wherein the inducible regulatory T cells have at least one characteristic selected from the group consisting of CTLA4 positive, NT5E positive, ITGAE (CD103) positive, and AREG positive.

17. The pharmaceutical composition of any one of claims 14 to 16, wherein the inducible regulatory T cells have at least two characteristics selected from the group consisting of CTLA4 positive, NT5E positive, ITGAE (CD103) positive, and AREG positive.

18. The pharmaceutical composition of any one of claims 14 to 17, wherein the inducible regulatory T cells are at least CTLA4 positive.

19. The pharmaceutical composition of any one of claim 14 to 18, wherein the CNS2 site of the FOXP3 gene of the inducible regulatory T cells are demethylated.

20. The pharmaceutical composition of any one of claim 14 to 19, wherein the inducible regulatory T cells are CD4 positive or CD8 positive.

21. The pharmaceutical composition of any one of claim 14 to 20, wherein the inducible regulatory T cells are obtained from or induced from human peripheral blood T cells or human tissue-derived T cells.

22. The pharmaceutical composition of any one of claims 14 to 21, wherein
the inducible regulatory T cells are obtained by a method including:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

23. The pharmaceutical composition of any one of claim 14 to 22, comprising a T cell population wherein about 50% or more of the cells in the T cell population are the inducible regulatory T cells.

24. The pharmaceutical composition of any one of claim 14 to 23, comprising a T cell population wherein about 80% or more of the cells in the T cell population are the inducible regulatory T cells.

25. The pharmaceutical composition of claim 23 or 24, wherein the T cell population is a regulatory T cell population.

26. The pharmaceutical composition of any one of claims 23 to 25, wherein about 90% or more of the cell population is T cells.

27. The pharmaceutical composition of any one of claims 14 to 26, comprising the inducible regulatory T cells such that about 10⁸ to about 10⁹, or about 10⁷ cells/kg are administered per dose.

28. The pharmaceutical composition of any one of claims 14 to 27 for treating or preventing a T cell-associated disease.

29. The pharmaceutical composition of claim 28, wherein the T cell-associated disease comprises an autoimmune disease, allergy, graft rejection, a graft versus host disease, an inflammatory disease, an infection, cancer, and ALS.

30. The pharmaceutical composition of claim 29, wherein the autoimmune disease comprises systemic lupus erythematosus, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, ulcerative colitis, and cardiomyopathy.

31. The pharmaceutical composition of any one of claims 14 to 30, wherein the pharmaceutical composition is administered by injection.

32. The pharmaceutical composition of any one of claims 14 to 31, wherein when the pharmaceutical composition is administered to a patient, the pharmaceutical composition is additionally administered to a patient who exhibits no response or an insufficient response to the pharmaceutical composition.

33. The pharmaceutical composition of claim 32, wherein the pharmaceutical composition is additionally administered at least about 2 weeks after the first administration.

34. A therapeutic agent for a T-cell associated disease comprising inducible regulatory T cells of any one of claim 1 to 9 or a cell population of any one of claim 10 to 13, wherein a diagnosis is made with respect to the T cell-associated disease in a diseased subject, and an appropriate CAR contained in the inducible regulatory T cells or the cell population is selected based on the diagnosis.

35. A method of producing inducible regulatory T cells containing a chimeric antigen receptor (CAR), the method comprising:
(a) a step of stimulating CD4-positive T cells or CD8-positive T cells in peripheral blood with a first basal medium for about 1 to about 5 days;
(b) a step of dormant culturing cells obtained in step (a) in a medium including IL-2 for at least about 1 to about 3 days;
(c) a step of stimulating cells obtained in step (b) with a second basal medium for about 1 to about 5 days;
(d) a step of dormant culturing cells obtained in step (c) in a medium including IL-2 for at least about 1 to about 3 days; and
(e) a step of introducing the CAR gene at least once in any of steps (a) to (d).

36. The method according to claim 35, wherein the introduction of the CAR gene is performed at least about twice in total in any of steps (a) to (d).

37. The method according to claim 35 or 36, wherein the introduction of the CAR gene is performed at least once in step (a).

38. The method of any one of claims 35 to 37, wherein the first basal medium comprises at least one factor selected from the group consisting of an anti-CD3 antibody, TGF-β1, IL-2, retinoic acid, a CDK8 inhibitor, a CDK19 inhibitor, a CDK8/19 inhibitor, and ascorbic acid.

39. The method of any one of claims 35 to 38, wherein the first basal medium comprises an anti-CD3 antibody, TGF-β1, IL-2, retinoic acid, a CDK8 inhibitor, a CDK19 inhibitor, a CDK8/19 inhibitor, and ascorbic acid.

40. The method of any one of claims 35 to 39, wherein the second basal medium comprises at least one factor selected from the group consisting of an anti-CD3 antibody, TGF-β1, IL-2, retinoic acid, a CDK8 inhibitor, a CDK19 inhibitor, and a CDK8/19 inhibitor.

41. The method of any one of claims 35 to 40, wherein the second basal medium comprises an anti-CD3 antibody, TGF-β1, IL-2, retinoic acid, a CDK8 inhibitor, a CDK19 inhibitor, and a CDK8/19 inhibitor.

42. The method of any one of claim 35 to 41, wherein step (a) stimulates the CD4-positive T cells or CD8-positive T cells with the first basal medium for about 3 days.

43. The method of any one of claims 35 to 42, wherein step (b) comprises dormant culturing the cells obtained in step (a) in the medium including IL-2 for at least about 2 days.

44. The method of any one of claims 35 to 43, wherein step (c) stimulates the cells obtained in step (b) with the second basal medium for about 3 days.

45. The method of any one of claims 35 to 44, wherein step (d) comprises dormant culturing the cells obtained in step (c) in the medium including IL-2 for at least about 2 days.

46. Inducible regulatory T cells or a cell population comprising the inducible regulatory T cells generated by the method of any one of claim 35 to 45.
